# EUROPEAN PATENT SPECIFICATION

(11) **EP 2 593 088 B1**
(45) Date of publication and mention of the grant of the patent: **07.01.2015**
(21) Application number: 11736192.3
(22) Date of filing: 14.07.2011
(51) Int. Cl.: A61K 9/16, A61K 9/51, A61K 38/17

(54) **MICROPARTICLES COMPRISING A SMALL HEAT-SHOCK PROTEIN**
MIKROPARTIKEL MIT EINEM KLEINEN HITZESCHOCKPROTEIN
MICROPARTICULES COMPRENANT UNE PETITE PROTÉINE DE CHOC THERMIQUE

(30) Priority: 16.07.2010 EP 10169869
(43) Date of publication of application: 22.05.2013
(73) Proprietor: Delta Crystallon B.V., 1951 NH Velsen-Noord (NL)
(72) Inventor: VAN NOORT, Johannes Maria, NL-2334 ER Leiden (NL)
(74) Representative: Jansen, Cornelis Marinus
(86) International application number: PCT/NL2011/050510
(87) International publication number: WO 2012/008834

(56) References cited:
- WO-A2-2007/022140
- WO-A2-2008/073466
- LEE J ET AL: "Controlled delivery of heat shock protein using an injectable microsphere/hydrogel combination system for the treatment of myocardial infarction", JOURNAL OF CONTROLLED RELEASE, ELSEVIER, AMSTERDAM, NL, vol. 137, no. 3, 4 August 2009 (2009-08-04), pages 196-202, XP026223531, ISSN: 0168-3659, DOI: DOI:10.1016/J.JCONREL.2009.04.008 [retrieved on 2009-04-14]
- TAYLOR R P ET AL: "Small heat shock proteins: a new classification scheme in mammals", JOURNAL OF MOLECULAR AND CELLULAR CARDIOLOGY, ACADEMIC PRESS, GB, vol. 38, no. 3, 1 March 2005 (2005-03-01), pages 433-444, XP004762353, ISSN: 0022-2828, DOI: DOI:10.1016/J.YJMCC.2004.12.014

## Description

### FIELD OF THE INVENTION

The present invention is in the field of medicine. In particular, it is in the field of medicaments used in the treatment of inflammatory diseases.

### BACKGROUND OF THE INVENTION

The small heat-shock protein family is characterized by a common characteristic which is the presence of the highly conserved so-called alpha crystallin domain comprising 90- 100 residues. The vertebrate eye lens proteins-alpha A- and alpha B-crystallin-and the ubiquitous group of 15-30-kDa heat-shock proteins, including HSPB1, HSPB2, HSPB3, HSPB6, HSPB7, HSPB8, HSPB9 and HSPB10 belong to this group of small heat-shock proteins. The two subunits of eye lens alpha-crystallins are alpha A-crystallin (CRYAA) and alpha B-crystallin (CRYAB). While CRYAA is preferentially expressed in the eye lens, CRYAB is expressed widely in many tissues and organs. The primordial role of the small heat-shock proteins is believed to be to counteract the destabilizing effects of stressful conditions on cellular integrity. There is evidence that they are involved, inter alia, in degenerative diseases.

Alpha-crystallins have been described as potential medicaments in a number of diseases and disorders. In WO2008073466 a method is described for inhibiting an inflammatory disease in a patient, comprising administering to a patient a therapeutically effective dose of free soluble CRYAB protein, wherein immune cells in tissues affected by the autoimmune disease have decreased activation in the presence of the agent. In WO9533997, the medical use of free soluble CRYAB protein in multiple sclerosis is described.

A drawback of proteins of the alpha-crystallin family is that these products as free soluble proteins are less effective than expected, especially when administered to humans. The goal of the invention is to solve this problem.

### SUMMARY OF THE INVENTION

The invention is based on the finding that small heat-shock proteins are able to activate macrophages far more effectively when administered in the form of biodegradable microparticles in comparison to their administration in free soluble form. Hence, the small heat-shock proteins in aspects of this invention are not administered as free soluble proteins.

The invention therefore provides a biodegradable microparticle having a diameter between 0.2 and 3.5 micrometer and comprising a pharmaceutically effective amount of at least one small heat-shock protein that induces IL-10 production in macrophages, said small heat-shock protein comprising an amino acid sequence identity of at least 50% to any of the sequences listed as SEQ ID NOs:1 and 12-26, or a combination thereof. The amino acid sequence identity of at least 50% to any of the sequences listed as SEQ ID NOs:1 and 12-26 indicates that the small heat-shock protein comprised an alpha-crystallin domain. Such an alpha-crystallin domain is the active region of the protein that determines whether it activates macrophages, which activation becomes apparent by the induction of IL-10 production in the macrophage. The alpha-crystallin domain may have an amino acid sequence identity of at least 50%, preferably at least 55%, more preferably at least 60%, 70%, 80%, 90% or 95% to SEQ ID NOs: 1 and 12-26. Preferably, said small heat-shock protein is the protein with the amino acid sequence selected from the group of SEQ ID NOs: 2-11. More preferably, said small heat-shock protein is the protein with the amino acid sequence of SEQ ID NO: 2.

In preferred embodiments, said biodegradable microparticle is biocompatible. In other preferred embodiments, said microparticle comprises capralactone, polylactide (PLA), polylactic-co-glycolic (PLGA) or polylactic-co-hydroxymethylglycolic acid (PLHMGA). Preferably, the biodegredable microparticles have a mean diameter between 0.2 and 3.5 µm.

The invention further provides the biodegradable microparticle according to the invention for use in a medical treatment of a subject. Preferably, said subject is a human subject. Preferably, said medical treatment is directed to an inflammatory disease. Preferably, said inflammatory disease is an acute or chronic inflammatory disorder of the skin, mucosa, the lungs, the nervous system the vascular system, the pancreas or of a joint, preferably dermatitis, psoriasis, eczema, Crohn's disease, ulcerative colitis, paradontitis, lichen planus, lichen sclerosus, chronic obstructive pulmonary disorder, emphysema, Alzheimer disease, Parkinson disease, dementia, optic neuritis, encephalitis, inflammatory peripheral neuropathies, atherosclerosis, vasculitis, rheumatoid arthritis or diabetes.

The invention further provides a pharmaceutical composition comprising a therapeutically effective dose of the biodegradable microparticle according to the invention. Preferably, at least 50, 60, 70, 80 or 90 percent of the microparticles present in the pharmaceutical composition are biodegradable microparticles according to the invention.

The invention further provides a method for producing a biodegradable microparticle according to the invention, comprising steps of mixing an aqueous solution comprising CRYAB with a solution of caprolactone, PLA, PLGA or PLHMGA in a volatile organic solvent, preferably dichloromethane (DCM) to provide a water/ volatile organic solvent two phase system; emulsifying said water/ volatile organic solvent two phase system to provide a water-in-oil emulsion; adding said water-in-oil emulsion to an aqueous solution comprising polyvinyl alcohol and emulsifying the resulting mixture to provide a water-in-oil-in-water emulsion; allow the volatile organic solvent to evaporate from said water-in-oil-in-water emulsion and allow the formation of biodegradable microparticles during said evaporation.

The invention further provides a method for treating a subject suffering from an inflammatory disease comprising administering to said subject a therapeutically effective amount of a biodegradable microparticle according to the invention or a pharmaceutical composition according to the invention.

### SHORT DESCRIPTION OF THE DRAWINGS

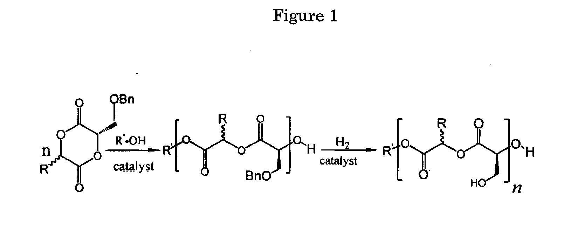

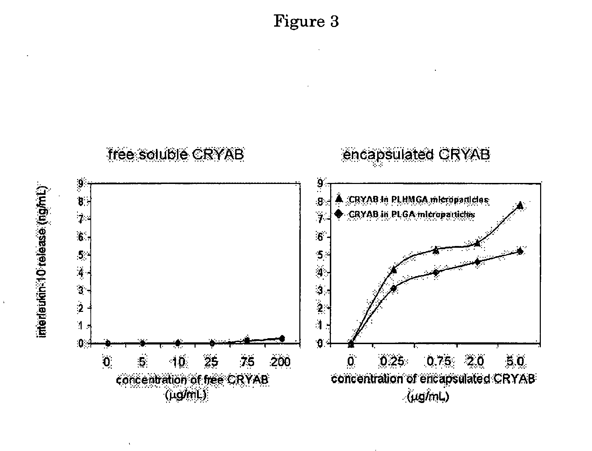

**Figure 1** **shows the synthesis of hydrophilic polyesters based on lactic acid and glycolic acid with pendant hydroxyl groups.**
   The reaction scheme in Fig. 1 illustrates the key step in the synthesis of hydrophilic polyesters which can be used to create microspheres that are more hydrophilic that the traditional poly (lactic co-glycolic acid) polymers. The preparation of such microspheres is described in more detail by Ghassemi et al. [J. Control. Release 138: 57-63 (2009)]. R= CH₃ in the BMMG monomer.

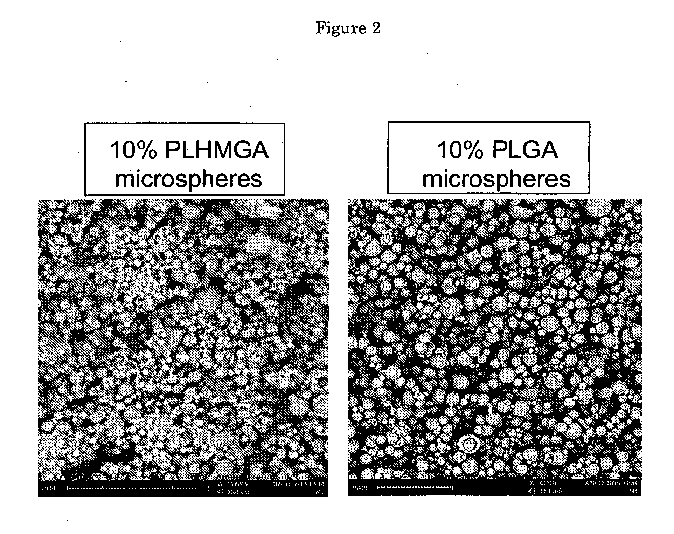

**Figure 2** **shows scanning electron micrographs of CRYAB-containing microspheres, based on either hydrophilic PLHMGA polymers, or PLGA polymers.**
   The images in Fig. 2 illustrate the similar size distribution of the microparticles obtained with either the traditional poly (lactic co-glycolic acid) (PLGA) polymers, or the more hydrophilic version containing hydroxymethylated polyesters (PLHMGA). While the diameter of PLGA microspheres, prepared as described in more detail in the examples, is generally between 0.2 and 3.5 micrometers, the diameter of PLHMGA microspheres prepared in a similar way is generally 0.2 to 2 micrometers.
**Figure 3** **shows the induction of IL-10 by free soluble CRYAB (left) and microsphere-encapsulated CRYAB (right).**
   The data in Figure 3 illustrate that the induction of IL-10 by macrophages by microsphere-encapsulated CRYAB is far more effective than if macrophages are exposed to free soluble CRYAB. It further shows that PLHMGA microparticles are even more effective than PLGA microparticles.
**Figure 4** **shows the sequence alignment of the ten family members of alpha-crystallin/small heat shock proteins. The boxed sequences represent the conserved alpha crystallin-like domain [adapted from** Kappé et al. (2003) Cell Stress Chaperones 8: 53**].**

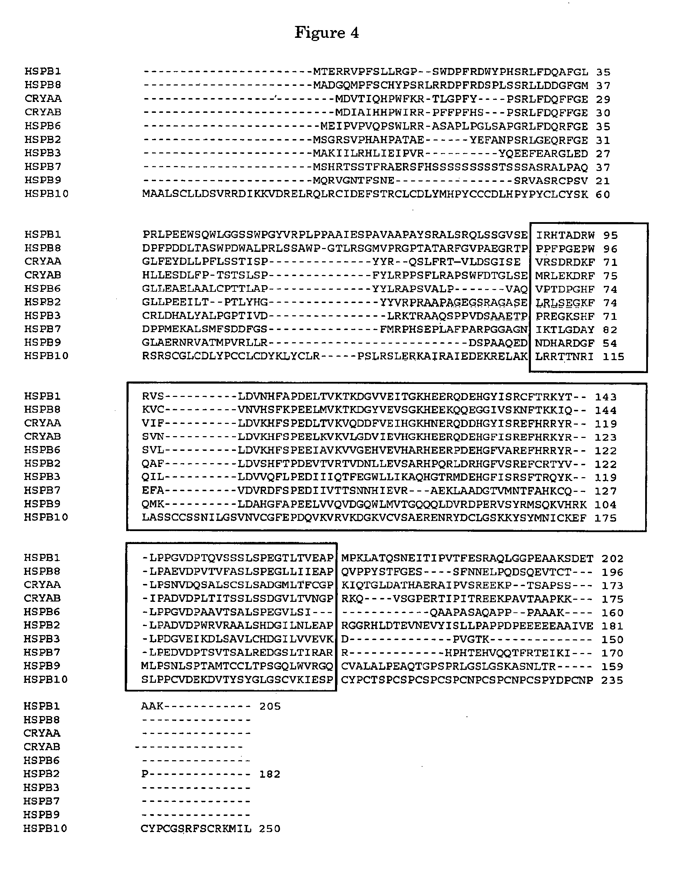

   Figure 4 shows a sequence alignment of all currently known ten alpha-crystallin/small heat shock proteins, highlighting the protein segments of marked homology known as the alpha-crystallin domain.

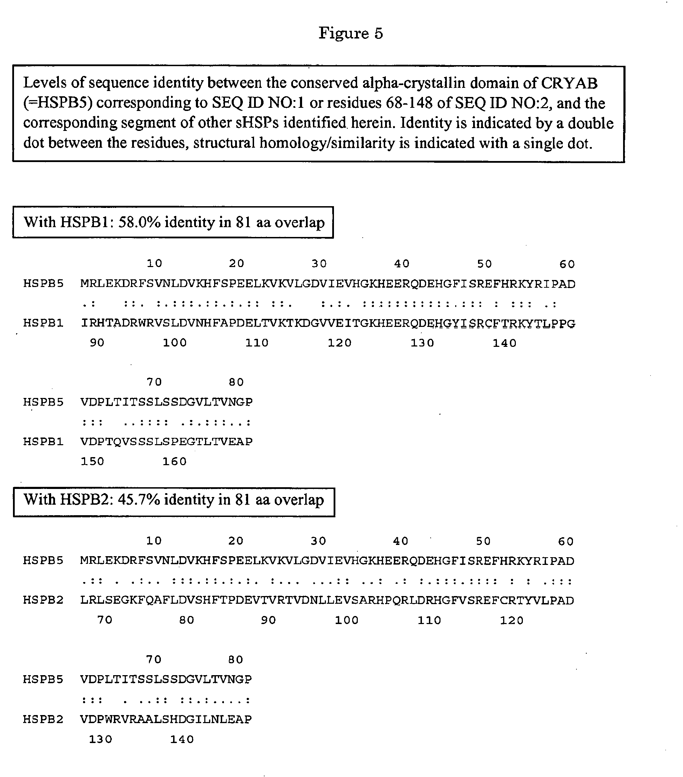

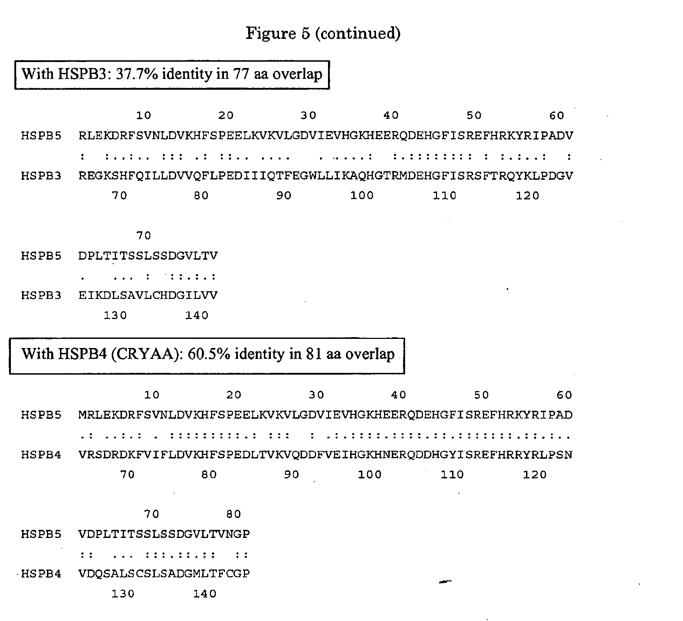

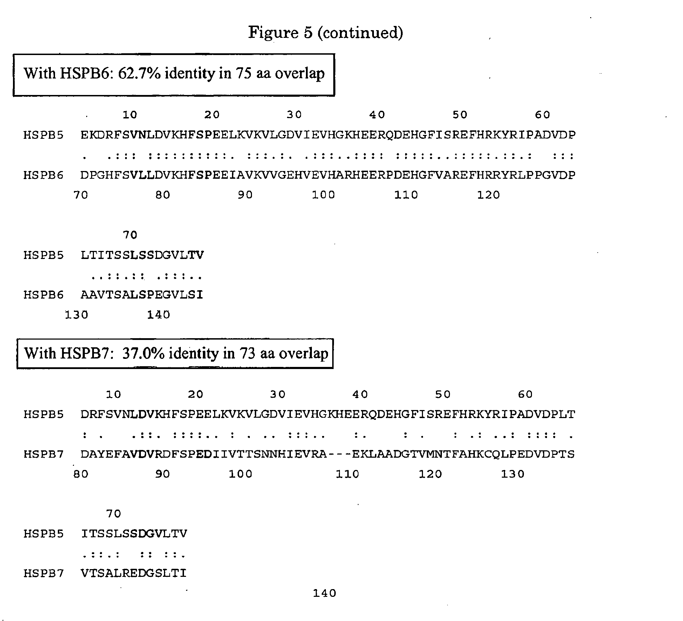

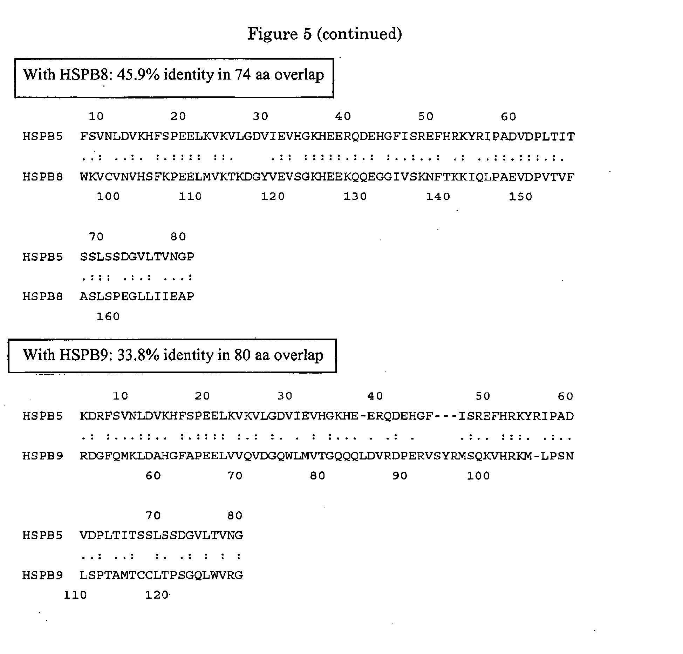

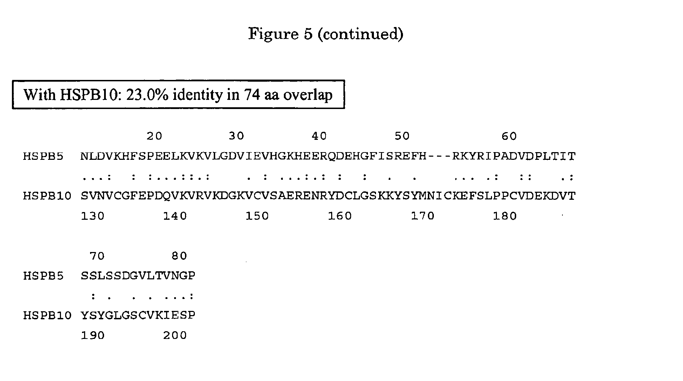

**Figure 5** **shows the homologies between the alpha-crystallin domain of CRYAB (residues 68-148) and the alpha-crystallin domains of other small heat shock proteins.**
   Sequence identity is indicated by a double dot, and structural homology by a single dot positioned between residues. This figure shows in more detail the extent of sequence identity and structural homology among the ten different human small heat-shock proteins.

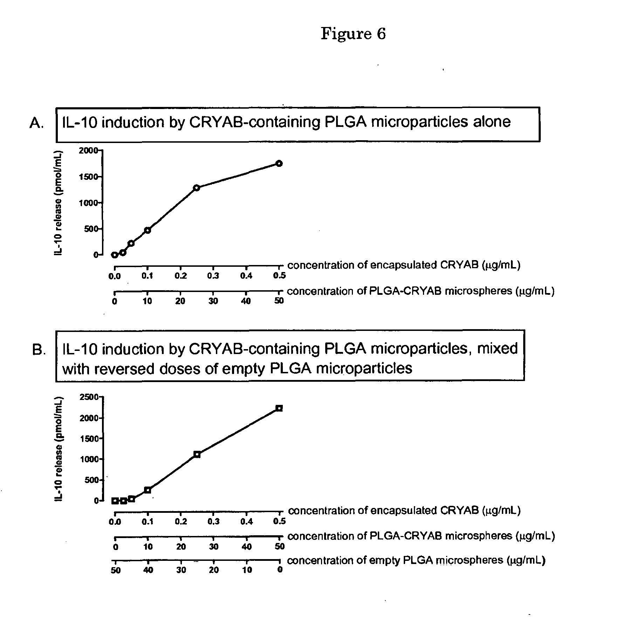

**Fig. 6** **illustrates that empty PLGA microspheres do not only fail to induce IL-10 production by human macrophages, but also do not influence IL-10 production when it is induced by CRYAB-containing microspheres.**
   When decreasing concentrations of empty microspheres are added to a culture of human macrophages to complement increasing concentrations of CRYAB-containing microspheres to a constant level of total microspheres, the response profile is the same as the one obtained with increasing concentrations of CRYAB-containing microspheres only. This confirms that the macrophage response to PLGA-microspheres which contain CRYAB is indeed mediated by the encapsulated protein, and not by microspheres as such. Empty PLGA microspheres of the same size and chemical characteristics not only fail to induce any production, they also do not influence the induction of IL-10 by CRYAB-containing microspheres.

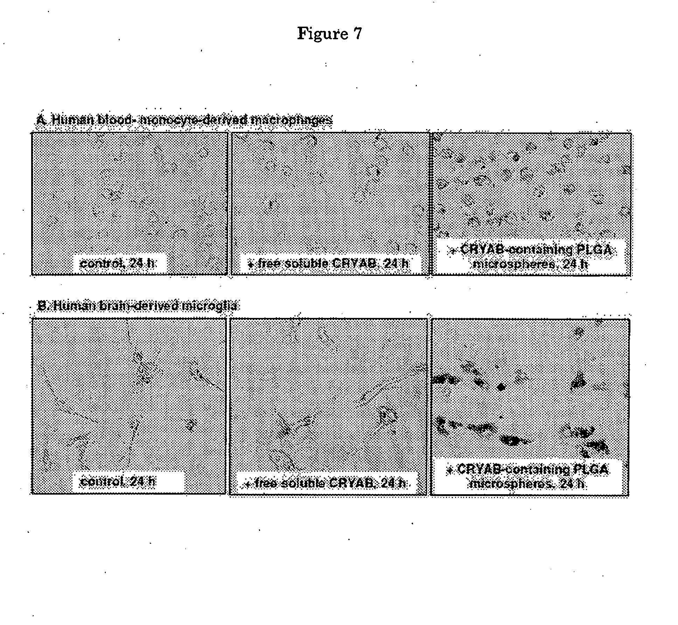

**Fig. 7** **illustrates rapid and complete phagocytosis of multiple CRYAB-containing microspheres by human blood monocyte-derived macrophages, and by human brain-derived microglia.**
   Different from most applications sought for microspheres, the current invention is not aimed at slow release of the therapeutic protein from microspheres over days to weeks. Instead, rapid uptake of CRYAB-containing microspheres by macrophages is aimed for, followed by rapid release of the therapeutic protein inside phagosomes. Fig. 3 shows how this strategy leads to marked production of the anti-inflammatory factor IL-10 in human macrophages within a 20 h timeframe. In Fig. 7, it is further illustrated how the currently described CRYAB-containing microspheres are rapidly and essentially completely phagocytosed by different types of human macrophages. In the right hand panels, macrophages and microglia are shown which have taken up multiple microspheres per cell within a 24-h timeframe. Cells cultured during this time without any addition, or cells supplied with free soluble CRYAB are shown for comparison.

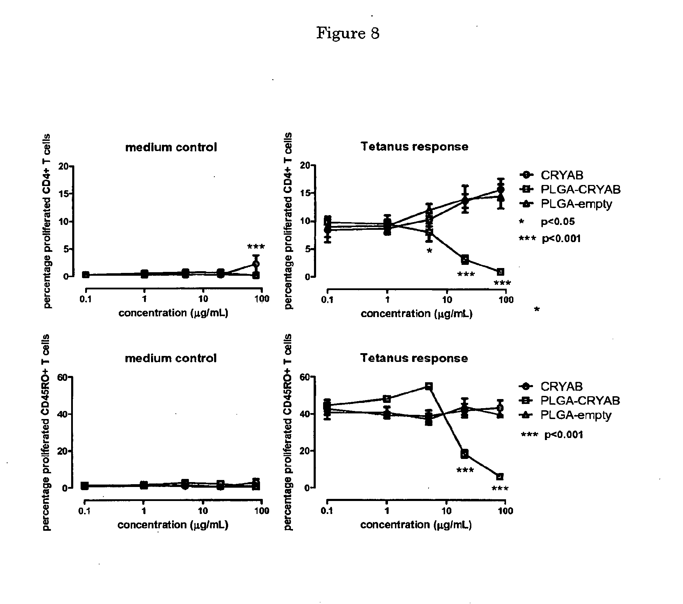

**Fig. 8** **illustrates the anti-inflammatory quality of the human immune response induced by PLGA microspheres containing CRYAB.**
   Different from other mammals, the adult human immune system contains memory T-cells that are responsive to CRYAB, along with serum antibodies against CRYAB. This immune responsiveness is primed through natural processes, and is found in all humans. The drawback resulting from this condition is that free soluble CRYAB will not only activate macrophages in humans, but can also activate memory T-cells, which will contribute to inflammation, rather than help dampen it. Fig. 8 illustrates that free soluble CRYAB indeed induces an antigen-specific T cell response in cell culture (left hand panels; top panels are for CD4⁺ helper T cells, bottom panels for CD45RO⁺ memory T cells), As a consequence, free soluble CRYAB is ineffective in suppressing the T cell response to another antigen, in this case tetanus toxoid (right hand panels). If fact, the addition of free soluble CRYAB to a cell culture of peripheral blood mononuclear cells leads to an increase in T-cell responses. In contrast, CRYAB-containing microspheres, but not empty microspheres, strongly suppress the T-cell response to tetanus toxoid, emphasizing their anti-inflammatory effect. In contrast to free soluble CRYAB, therefore, CRYAB-containing microspheres activate an anti-inflammatory response by macrophages, without triggering a pro-inflammatory response by (memory) T cells.

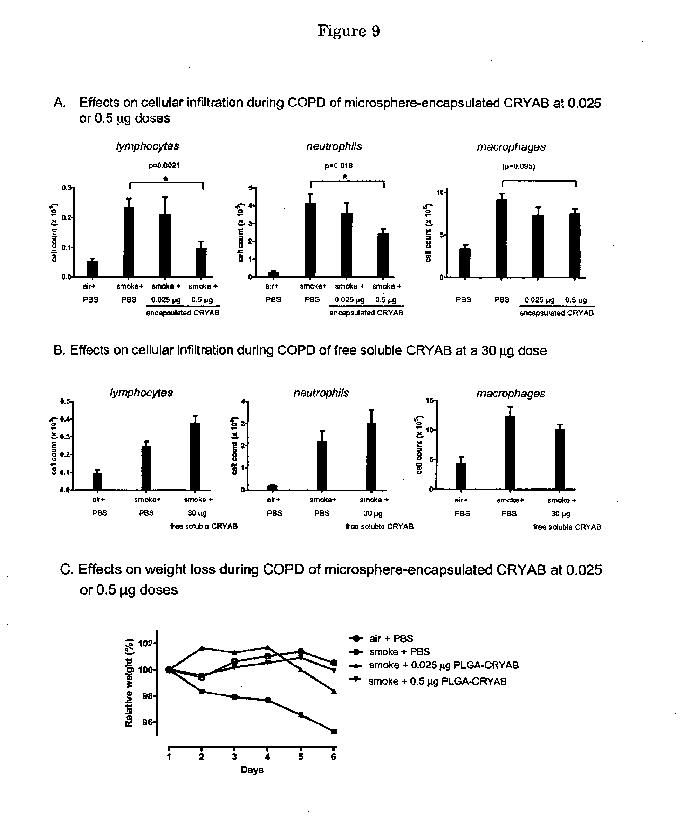

**Fig. 9** **shows the therapeutic anti-inflammatory activity of CRYAB-loaded PLGA microspheres in a mouse model for chronic obstructive pulmonary disorders (COPD).**
   The therapeutic anti-inflammatory activity of CRYAB-containing microspheres was demonstrated by treatment of cigarette-smoke induced inflammation in a mouse model for COPD. As the result of cigarette-smoke induced lung inflammation, significant numbers of lymphocytes, macrophages, and neutrophils infiltrate the lungs over a period of 6 days. Treatment twice a day with CRYAB-containing microspheres, starting after the first exposure to smoke, led to a marked and statistically significant suppression of lymphocyte and neutrophil recruitment, reducing the numbers of these infiltrated cells by 75% and 44%, respectively (Fig. 9A). Reduction of macrophage numbers was markedly more modest, and did not reach levels of statistical significance. In contrast, free soluble CRYAB at a comparable or even much higher dose was unable to exert such a therapeutic anti-inflammatory effect (Fig. 9B). In addition to the therapeutic inhibitory effect on cellular infiltration, weight loss of the animals which is normally seen during COPD, was almost completely prevented by micropshere-encapsulated CRYAB (Fig. 9C).

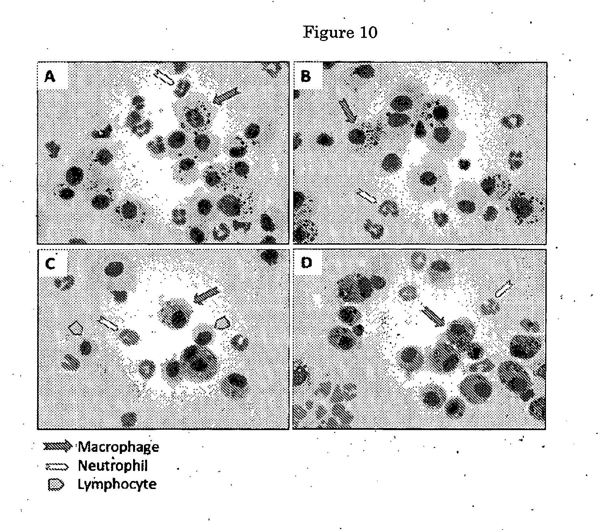

**Fig. 10** **shows the haematoxilin-eosin staining of cells collected from broncho-alveolar lavages after CRYAB-containing PLGA microspheres were intratracheally administered to smoke-exposed mice.**
   The Figure shows the selective uptake of CRYAB-containing microspheres by alveolar macrophages only, following intratracheal administration to mice. Following therapeutic treatment of mice with the microspheres for a period of 5 days to suppress smoke-induced inflammation, all cells were harvested from the lungs by broncho-alveolar lavage. In the population of cells thus obtained, macrophages, lymphocytes and neutrophils can be readily identified on the basis of their morphology. Also microspheres are easily identified by their dark appearance and size. As shown in this Figure, only large, activated macrophages contain phagocytosed microspheres, while neutrophils and lymphocytes do not. This confirms that the therapeutic effect of the CRYAB-containing PLGA microspheres is exclusively mediated by the macrophage response to these microparticles.

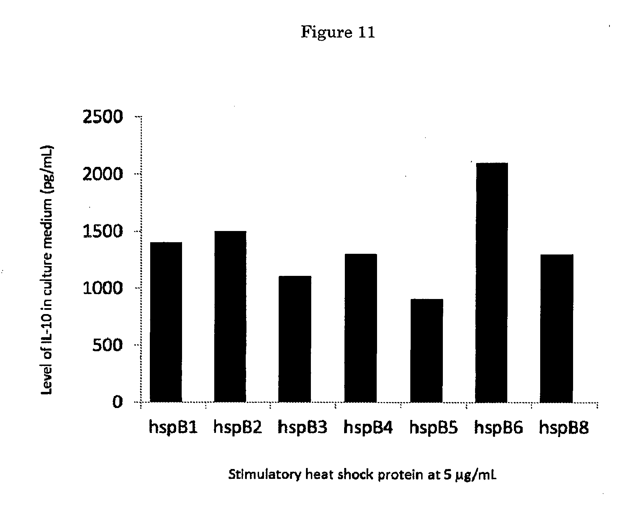

**Fig. 11** **shows the induction of interleukin-10 in human monocyte-derived macrophages by different members of the family of small heat shock proteins as defined herein. Background levels of IL-10 were subtracted from all values.**
   The Figure shows that apart from CRYAB, also other members of the family of small heat shock proteins have the ability to induce production of the powerful immune-regulatory factor IL-10 by human macrophages. Recombinant, purified heat shock proteins as indicated were added at a concentration of 5 µg/mL to cultures of differentiated human macrophages, and levels of IL-10 appearing in the culture medium were quantitated as described before. Measurements were performed in duplicate. The result as shown this Figure confirms that heat-shock protein family members of CRYAB not only share the conserved alpha crystallin domain, but because of it, also the ability to activate macrophages.

### DETAILED DESCRIPTION OF THE INVENTION

### Definitions

The term "microparticle" as used herein encompasses "nanoparticles", "microcapsules", "microbeads", and "microspheres". It is essential that the microparticle as used herein is a biodegradable particle which is smaller than 3.5 µm and larger than 0.2 µm, preferably smaller than 2 µm and larger than 1 µm. The size of a microparticle as specified herein refers to the mean particle diameter. The size of the microparticle is important because the microparticle must be phagocytosed by phagocytes in order to activate the phagocytes through the release of the small heat shock proteins as indicated herein. Activation of the macrophages becomes apparent from the induction of IL-10 production by the macrophage.

The microparticle may have any form, including a substantially spherical and irregular form. If a microparticle is not spherical, the term diameter refers to the inner diameter of the smallest spherical structure wherein said microparticle would fit. A microparticle can be a homogeneous microparticle. The term "homogeneous microparticle" as used herein refers to a microparticle having its active agent (i.e. alpha-crystallin) dispersed or dissolved throughout the microparticle. Homogeneous microparticles are preferably structurally formed by a matrix of an excipient, usually a polymeric excipient. Preferably, in homogeneous microparticles, said polymeric excipient is a biodegradable polymer. Preferably, said biodegradable polymer is present throughout each homogeneous microparticle, with the active agent captured within the biodegradable polymer molecules. Said polymeric excipient may be of the same polymer or contain a mix of different types of polymers.

Other microparticles which may be used in aspects of the invention are encapsulating microparticles.

The term "encapsulating microparticle" as used herein refers to a microparticle which comprises a biodegradable coating encapsulating a composition containing the agent or the agent in a substantially pure form. The agent may be dispersed or dissolved throughout said composition. The outer membrane of the encapsulating microparticle, which has a function of delaying the release of said agent, preferably comprises or consists of a biodegradable polymer.

The term "biodegradable microparticle" as used herein refers to the capacity of a microparticle to be broken down into smaller fragments or to release an active agent over time under physiological conditions. Degradation may occur, for example, by enzymatic, chemical or physical processes. Biodegradable microparticles typically release their agent via a combination of drug diffusion and polymer erosion. Preferably, such smaller fragments are smaller than 90, 80, 70, 60, 50, 40, 30, 20, 10, 5, 4, 3, 2, 1% of the biodegradable microparticle diameter which the microparticle had before it was exposed to a fluid under physiological conditions. In preferred embodiments of biodegradable microparticles, a smaller fragment refers to fragments smaller than 50, 40, 30, 20, 10 nm.

The term "physiological condition" refers a condition as present in a biological system. Preferably it refers to a possible value of a parameter of a fluid to which the microparticle is exposed, which is considered physiological if the parameter has a certain value which occur in a tissue or bodily fluid of a living warm blooded vertebrate animal. Preferably, said parameter comprises the temperature, sodium concentration, hydrostatic pressure, osmotic pressure, and/or pH. Preferably, "under physiological conditions" means that at least the temperature, hydrostatic pressure, osmotic pressure, and pH of said fluid are within the range of values as they normally are present in a tissue or bodily fluid of a living warm blooded vertebrate animal. Preferably, said physiological conditions include a temperature between 35 and 41 degrees, and a pH between 2 and 9, more preferably between 7 and 8 and even more preferably between 7.35 and 7.45. In alternative preferred embodiments, the physiological conditions refer to the values of a parameter as they are present within the endosomal and/or phagosomal compartments of a macrophage, involving a decreased pH, preferably between 5 and 7. More preferably, it refers to a possible value of a parameter as it is present in blood, a white blood cell, most preferably a macrophage. In preferred embodiments the term biodegradable means that a microparticle is degraded when taken up by a macrophage. More preferably, said microparticle is degraded within a macrophage at a higher rate than in a body fluid, preferably blood.

The term "over time" as used herein means within a year, but more preferably within a month, a week, a day, or an hour.

The term "biodegradable polymer" as used herein refers to a polymer which is degraded over time under physiological conditions as described above. Examples of biodegradable polymers include those having at least some repeating units representative of at least one of the following: an alpha-hydroxycarboxylic acid, a cyclic diester of an alpha-hydroxycarboxylic acid, a dioxanone, a lactone, a cyclic carbonate, a cyclic oxalate, an epoxide, a glycol, and anhydrides. Preferred biodegradable polymers comprise at least some repeating units representative of polymerizing at least one of lactic acid, glycolic acid, lactide, glycolide, ethylene oxide and ethylene glycol.

Preferred biodegradable polymers include poly(lactide)s, poly(glycolide)s, poly(lactic acid)s, poly(glycolic acid)s, polyanhydrides, polyorthoesters, polyetheresters, polycaprolactone, polyesteramides, polycarbonate, polycyanoacrylate, polyurethanes, polyacrylate, blends and copolymers thereof.

The range of molecular weights contemplated for the polymers to be used in the present processes can be readily determined by a person skilled in the art based upon factors such as the desired polymer degradation rate, or preferably the level macrophage activation under (simulated) in vivo conditions, preferably in humans. Typically, the range of molecular weight is between 2000 to 2,000,000 Daltons.

Preferred polymers are selected from ε̇-caprolactone, polylactic acid (PLA), polylactic-co-glycolic acid (PLGA) and polylactic-co-hydroxymethylglycolic acid (PLHMGA). Preferably these polymers form the matrix material of the microparticle according to the invention.

The term "a (co)polymer of lactic acid and/or glycolic acid" as used herein is intended to refer to a polymer of lactic acid alone, a polymer of glycolic acid alone, a mixture of such polymers, a copolymer of glycolic acid and lactic acid, a mixture of such copolymers, or a mixture of such polymers and copolymers.

The term "biocompatible polymers" refers to biocompatible polymers that degrade to nontoxic products. Specific examples of biocompatible polymers that degrade to nontoxic products that do not need removal from biological systems include poly(hydro acids), poly (L-lactic acid), poly (D,L-lactic acid), poly (glycolic acid) and copolymers thereof.

The term "biocompatible microparticle" as used herein refers to a microparticle which has no toxic or injurious effects on biological systems. In a preferred embodiment of the biodegradable microparticle it refers to a microparticle which is able to perform its desired function with respect to a medical therapy, without eliciting any undesirable local or systemic effects in the recipient or beneficiary of that therapy, but generating an appropriate beneficial cellular or tissue response in that specific situation, and preferably optimizing the clinically relevant performance of that therapy.

The term "drug delivery device" as used herein refers to a microparticle without an active agent. Many types of microparticles have been described in the prior art, or methods for producing such microparticles, sometimes specifying the inclusion of a specific active agent. If reference is made herein to a certain microparticle of a particular prior art document, it is meant that reference is made to the microparticle without the active agent of the prior art, in case that the prior art document specifies the inclusion of a specific active agent, unless specified otherwise. In case reference is made to a method for producing a particular microparticle of the prior art wherein a specific active compound is included in that particular microparticle, it is meant that reference is made to the method wherein the active agent of the prior art is replaced by the small heat-shock protein as described herein.

The term "small heat-shock protein" is used herein to refer to a proteinaceous compound having an amino acid sequence identity of at least about 50%, preferably at least 56%, more preferably at least 60%, more preferably at least 70%, more preferably at least 80%, more preferably at least 85%, still more preferably at least 90%, even more preferably at least 95%, and particularly preferably at least 97%, 98% and most preferably at least 99%, with the alpha-crystallin domain of either one of the small heat-shock proteins HSPB1, HSPB2, HSPB3, HSPB4, HSPB5, HSPB6, HSPB7, HSPB8, HSPB9, or HSPB10 as indicated in Fig. 4 (SEQ ID NO: 2-11). Preferably the amino acid sequence identity relates to a region of at least 40 contiguous amino acids, more preferably at least 50, more preferably at least 60, more preferably at least 70, more preferably at least 73, more preferably at least 74, more preferably at least 75, more preferably at least 77, most preferably at least 80 contiguous amino acids. Preferably, said small heat-shock protein has an amino acid sequence having a sequence selected from the group of SEQ ID NOs: 2-11.

The term "amino acid sequence similarity" as used herein denotes the presence of similarity between two polypeptides or proteins. Polypeptides have "similar" sequences if the sequence of amino acids in the two sequences is the same when aligned for maximum correspondence. Sequence comparison between two or more polypeptides is generally performed by comparing portions of the two sequences over a comparison window to identify and compare local regions of sequence similarity. The comparison window is typically from about 10 to 80 contiguous amino acids. The "percentage of sequence similarity" for polypeptides, such as 50, 60, 70, 80, 90, 95, 98, 99 or 100 percent sequence identity may be determined by comparing two optimally aligned sequences over a comparison window, wherein the portion of the polypeptide sequence in the comparison window may include amino acid deletions, modification or addition of single amino acids or groups of amino acids as compared to the reference sequence (which does not comprise additions or deletions) for optimal alignment of the two sequences. The percentage is calculated by: (a) determining the number of positions at which the identical amino acid occurs in both sequences to yield the number of matched positions; (b) dividing the number of matched positions by the total number of positions in the window of comparison; and (c) multiplying the result by 100 to yield the percentage of sequence similarity. Optimal alignment of sequences for comparison may be conducted by computerized implementations of known algorithms, or by visual inspection. Sequence comparison and multiple sequence alignment algorithms are readily available on the internet, for instance William Pearson's "LALIGN" program. The LALIGN program implements the algorithm of Huang and Miller, published in Adv. Appl. Math. (1991) 12:337-357. It can be found at http://www.ch.embnet.org/software/LALIGN form.html.

The term "macrophage" comprises white blood cells within tissues, resulting from expansion and differentiation of monocytes. Typically, macrophages are about 21 micrometers in diameter. Macrophages are stationed at strategic points where microbial invasion or accumulation of dust is likely to occur. Macrophages in aspects of the invention include different types of macrophages, determined by their location in the body. These macrophages have specific names. Preferred macrophages include dust cells or alveolar macrophages which are located in the pulmonary alveolus of lungs, histiocytes located in connective tissue, Kupffer cells located in the liver, microglia located in neural tissue, epithelioid cells located in granulomas, osteoclasts located in bones, sinusoidal lining cells located in spleen and mesangial cells located in the kidney. Methods of identifying macrophages *in vitro* are well known to a skilled person and include the use of preferably monoclonal antibodies against membrane bound markers present on macrophages for identification. Preferred membrane markers comprise CD13, CD14 and CD68.

The term "activated macrophage" refers to a functional state of a macrophage, characterized amongst others by the expression levels of specific cytokines and/or chemokines. The term "activated macrophage" as used herein refers to macrophages characterized by an increase production of IL-10, TNF-a, CCL1, IL-13, CCL-5 and/or TGF-β relative to non-activated macrophages. Preferably, an activated macrophage does not express CCL18 or IL-12 at a significantly higher level than a normal macrophage.

The term "significantly higher" refers to a statistically different expression level, preferable at least 5-fold higher than unstimulated macrophages, preferably from the same subject. Preferably, said activation is further characterized by the presence of intracellular nitric oxide. Preferably, said activation is further characterized by the presence of MHC class II and CD86 surface markers on said macrophage. Preferably, said activated macrophage does not express CD80, CD163, FcyR or a mannose receptor.

Preferred methods of determining the levels of specific cytokines and/or chemokines associated with macrophage activation involve the use of commercially available enzyme-linked immunosorbent assays (ELISA) or PCR-amplification of transcripts of specific cytokines and/or chemokines. Preferably, the secretion of at least one of these of specific cytokines and/or chemokines is significantly higher in activated macrophages compared to secretion levels of unstimulated macrophages. Preferably, the secretion levels of said at least one cytokine is at least 5, 10, 15, 25, 50 or 100 higher when compared to unstimulated or non-activated macrophages, being macrophages from the same source, and cultured under identical conditions, but in the absence of the stimulus.

The term "activation of a macrophage" is used to designate a regulatory process wherein a macrophage undergoes physiological changes resulting in an activated form.

The term "inflammatory disease" refers to a pathological state of the body in which the activity of the immune system is pathologically stimulated or suppressed. In a preferred embodiment, said activity is the primary cause of the inflammatory disease. Preferably, said inflammatory disease is an acute or chronic inflammatory disorder of the skin, mucosa, the lungs, the nervous system the vascular system, the pancreas or of a joint, preferably dermatitis, psoriasis, eczema, Crohn's disease, ulcerative colitis, paradontitis, lichen planus, lichen sclerosis, chronic obstructive pulmonary disorder, emphysema, Alzheimer disease, Parkinson disease, dementia, optic neuritis, encephalitis, inflammatory peripheral neuropathies, atherosclerosis, vasculitis, rheumatoid arthritis or diabetes.

The term "pharmaceutically acceptable carrier" as used herein refers to a carrier for administration of said microparticle. The pharmaceutically acceptable carrier can comprise any substance or vehicle suitable for delivering said microparticle to a therapeutic target. The term refers to any pharmaceutical carrier that does not itself induce the production of antibodies harmful to the individual receiving the composition, and which may be administered without undue toxicity. Suitable carriers may be one or more optional stabilizers, diluents, or excipients.

As used herein, "a pharmaceutically effective amount" as used herein refers to an amount of the small heat-shock protein as described herein effective to elicit a detectable IL-10 level secreted by a macrophage. Preferably, the encapsulated protein at a stimulatory concentration of 1 µg/mL will induce accumulation of IL-10 to concentrations of at least 1 ng/mL over a 24-h period in a culture well containing between 100.000 and 150.000 human macrophages and a total medium volume of 200 µL.

The term "therapeutically effective amount" as used herein refers to the quantity of the biodegradable microparticle according to the invention necessary to prevent, cure or at least partially arrest the symptoms of the disorder and its complications. Amounts effective to achieve this goal will, of course, depend on the severity of the disease and the weight and general state of the patient. Typically, dosages used in vitro may provide useful guidance in the amounts useful for in situ administration of the pharmaceutical composition, and animal models may be used to determine effective dosages for treatment of particular disorders.

### Embodiments

The present invention is based on the surprising finding that microparticles containing CRYAB activate macrophages far more effectively than free soluble CRYAB.

The invention therefore provides a microparticle comprising CRYAA or CRYAB protein, preferably in a pharmaceutically effective amount. The activation of macrophages, leading to the production of the strongly anti-inflammatory substance IL-10, underlies the previously documented anti-inflammatory effects of CRYAB protein in different mouse models of inflammation.

### Microparticles

Microparticles according to the invention may be composed of various compositions and structures. Any biodegradable microparticle with a diameter between 0.2 and 3.5 micrometer may be used. Many processes for making drug delivery devices have been described which are suitable for preparing a microparticle according to the invention, by incorporating a pharmaceutically effective amount of a small heat-shock protein therein. In principle, any microparticle may be used if it is taken up by a macrophage and releases a pharmaceutically effective amount of a small heat-shock protein inside the macrophage. The effectiveness of microparticles according to the invention is *inter alia* related to their size. The microparticles must be phagocytosed by phagocytes. Therefore, preferred microparticles are equal in size or smaller than 3 µm. Such microparticles are suitable for oral or injectable delivery, for inhalation or for pulmonary delivery.

In a preferred embodiment, said microparticle has a mean diameter between 1 and 2.5 µm. In a preferred embodiment, the diameter of PLGA microparticles, prepared as described in more detail in the examples, is between 0.5 and 2 micrometers. Within this range the PLGA microparticles are very effective. With respect to the diameter of PLHMGA microparticles, which can be prepared in a similar way, the diameter is preferably between 0.2 to 2 micrometers to achieve good results.

Microparticles are preferably not liposomes. In certain embodiments, liposomes are explicitly disclaimed.

Suitable microparticles are nanoparticles made of Poly(ethylene oxide)-poly(L-lactic acid)/poly(e-benzyl-L-aspartate). Synthesis thereof is described in Figure 1 of Majeti N. V. Ravi Kumar in J Pharm Pharmaceut Sci 3(2):234-258, 2000. Also, nanoparticles, described in the same article as polyethylene glycol coated nanospheres, azidothymidin (AZT)/dideoxycytidine (ddc) nanoparticles, poly (isobutylcynoacrylate) nanocapsules, nanoparticles obtained from poly(y-benzyl-L-glutamate)/poly(ethylene oxide), chitosan-poly(ethylene oxide) nanoparticles and solid lipid nanoparticles are contemplated as a drug delivery devices for incorporating a pharmaceutically effective amount of a small heat-shock protein.

In addition, multiporous beads of chitosan, coated alginate microspheres, N-(aminoalkyl) chitosan microspheres, chitosan/calcium alginate beads, poly(adipic anhydride) microspheres, gellan-gum beads, poly(D, L-lactide-co-glycolide) microspheres, alginate-poly-L-lysine microcapsules, crosslinked chitosan microspheres, chitosan/gelatin microspheres, crosslinked chitosan network beads with spacer groups, 1,5-diozepan-2-one (DXO) and D,L-dilactide (D,L-LA) microspheres, triglyceride lipospheres, poly electrolyte complexes of sodium alginate chitosan, polypeptide microcapsules and albumin microspheres as described in Majeti N. V. Ravi Kumar in J Pharm Pharmaceut Sci 3(2):234-258, 2000, are contemplated as drug delivery devices for the small heat-shock protein as described herein.

Preferred encapsulated microparticles as drug delivery devices are described in US2004247670.

### Methods for preparing microparticles according to the invention

A wide variety of methods to prepare microparticles are described in the literature. Microparticles according to the invention can be made using any existing method. Suitable techniques include spray drying, milling or emulsion techniques. A suitable way of producing microparticles via milling is by cleaning sintered calcium phosphate mixed with the small heat-shock protein as described herein ultrasonically with acetone, ethanol and/or water, where after the microparticles are optionally dried and sterilized. A preferred way of preparation of said biodegradable microparticles is described in more detail by Ghassemi et al. [J Control. Release 138: 57-63 (2009)].

Suitable methods make use of emulsions to make biodegradable microparticles, in particular to make microparticles less than 100 µm in diameter. To give a general example of such processes, one can dissolve a polymer in a suitable organic solvent (the polymer solvent), dissolve or disperse an agent in this polymer solution, disperse the resulting polymer/agent mixture into an aqueous phase (the processing medium) to obtain an oil-in-water emulsion with oil micro droplets dispersed in the processing medium, and remove the solvent from the micro droplets to form microparticles. These processes can also be performed with water-in-oil emulsions and with double emulsions, i.e. water-in-oil-in-water emulsions.

The use of emulsion-based processes that follow this basic approach is described in several U.S. patents. For example, U.S. Pat. No. 4,384,975 describes the production of microparticles by forming an emulsion and then slowly removing the polymer solvent from the micro droplets in the emulsion by vacuum distillation. As another example, U.S. Pat. No. 3,891,570 discloses a method in which the polymer solvent is removed from the micro droplets in the emulsion by applying heat or reducing the pressure in the fabrication vessel. In still another example, U.S. Pat. No. 4,389,330, the polymer solvent is partially removed from the micro droplets in the emulsion by vacuum distillation (preferably 40 to 60% of the polymer solvent) and then the remainder of the polymer solvent is extracted to solidify the microparticles. The most widely used methods to prepare biodegradable microparticles are phase separation, spray drying, and solvent evaporation.

Phase separation, also known as coacervation, uses a decrease of the polymer solubility by the addition of a non-solvent. In a typical procedure, biodegradable polymer is dissolved in an organic solvent (e.g., dichloromethane). Lipophilic drugs are dissolved in the polymer solution. Hydrophilic drugs are dissolved in water and then dispersed in the polymer solution (water in oil (w/o) emulsion) or dispersed as a solid powder. A non-solvent (typically silicon oil) is gradually added. Two phases form: a polymer-rich silicon oil phase and a polymer-depleted liquid organic solvent phase. As the organic solvent is extracted or evaporates, polymer microparticles with entrapped drug solidify in the silicon oil phase. The coacervate (silicon oil) adsorbs to the polymer microparticles.

In spray drying, the biodegradable polymer is dissolved in volatile organic solvent, such as dichloromethane. The drug is dissolved or dispersed in the polymer solution. The solution or dispersion is sprayed in heated air. The solvent evaporates, resulting in the formation of solid microparticles.

Solvent evaporation is the most commonly used method of preparing microparticles. In this method a drug-containing organic polymer solution is emulsified into a dispersion medium that is typically aqueous but may be oil. The methods can be further classified into oil in water (o/w), water in oil in water (w/o/w), and oil in oil (o/o) emulsion methods.

In an o/w method, drug and polymer are dissolved in an organic solvent, such as dichloromethane or a methanol/dichloromethane mixture. The drug-polymer-organic solvent solution is dispersed in an aqueous phase. An emulsifier, typically poly(vinyl alcohol), is included in the aqueous phase to help form small organic solvent droplets in the aqueous phase. The organic solvent evaporates with stirring, and with the evaporation, the droplets solidify into polymer microparticles with entrapped drug.

In a w/o/w double emulsion, an aqueous drug solution is prepared and dispersed into a solution of the polymer in an organic solvent to form a water-in-oil emulsion containing the drug and polymer. The w/o polymer-drug emulsion is then emulsified into an aqueous phase to form a w/o/w emulsion. With stirring, the organic solvent evaporates, allowing the polymer-drug droplets in the emulsion to solidify into microparticles.

In an o/o emulsion method, drug and polymer are dissolved in a water-miscible solvent (e.g., acetonitrile). That solution is emulsified into an oily phase in the presence of an emulsifier such as SPAN 80 to form an oil-in-oil emulsion. The organic solvent is extracted by the oil and microparticles can be harvested by filtration.

In general, an aqueous solution, a suspension, or a solid form of the active agent can be admixed with the organic solvent containing the polymer. When an aqueous solution of active ingredient is used, polymer:active agent emulsions will be formed and used to prepare microparticles. When a suspension or solid form of active agent is used, polymer:active agent suspensions are formed and used to prepare the microparticles.

A preferred method for producing a microparticle according to the invention, comprises steps of adding a solution containing a small heat-shock protein as described herein to a solution of PLGA or PLHMGA in dichloromethane (DCM) resulting in a water/DCM two phase system, emulsifying said water/DCM two phase system resulting in a water-in-oil emulsion, adding a solution comprising polyvinyl alcohol resulting in a mixture, emulsifying said mixture resulting in a water-in-oil-in water emulsion, allow the DCM to evaporate from said water-in-oil-in water emulsion and collect the biodegradable microparticles.

### Treatment of inflammatory diseases

The biodegradable microparticles according to the invention very effectively activate macrophages. Therefore, they provide a much more efficient strategy to selectively deliver the small heat-shock protein as described herein to macrophages than simply supplying free soluble protein. Without wishing to be bound by theory, it is believed that this is because the microparticles are phagocytosed directly by their target cells. The biodegradable microparticles according to the invention are therefore suitable for use in a medical treatment of a human subject. The invention further provides a method for treating a human subject suffering from an inflammatory disease comprising administering to said human subject a microparticle according to the invention. The direct delivery of the microparticles will generally be accomplished by injection, either subcutaneously, intraperitoneally, intravenously or intramuscularly, or delivered to the interstitial space of a tissue. The microparticles can also be administered into the nervous system. Other modes of administration include topical, oral, suppositories, and transdermal applications, needles, and particle guns or hyposprays. Dosage treatment may be a single dose schedule or a multiple dose schedule. The biodegradable microparticles according to the invention may be administered on a daily, weekly or monthly basis.

Preferably, said biodegradable microparticle is used in a medical treatment directed to an inflammatory disease. Preferably, said inflammatory disease is an acute or chronic inflammatory disorder of the skin, mucosa, the lungs, the nervous system the vascular system, the pancreas or of a joint, preferably dermatitis, psoriasis, eczema, Crohn's disease, ulcerative colitis, paradontitis, lichen planus, lichen sclerosis, chronic obstructive pulmonary disorder, emphysema, Alzheimer disease, Parkinson disease, dementia, optic neuritis, encephalitis, inflammatory peripheral neuropathies, atherosclerosis, vasculitis, rheumatoid arthritis or diabetes.

### Pharmaceutical compositions

The invention further provides a pharmaceutical composition comprising an effective amount of microparticles. For purposes of the present invention, an effective dose will be from about 100 ng /kg to 50 mg/kg of the compositions in the individual to which it is administered. Alternatively, effective dose is expected to be in the range of 10 ng/mL to 10 mg/mL for topical applications.

Said pharmaceutical composition can also contain a pharmaceutically acceptable carrier. Pharmaceutically acceptable carriers are know in the art, and include, e.g., aqueous isotonic solutions for sterile injectable compositions, which can contain antioxidants, buffers, bacteriostats and solutes that render the formulation isotonic with the blood of the intended recipient, and aqueous and non-aqueous sterile suspensions, which can include suspending agents, solubilizers, thickening agents, stabilizers, preservatives, or microspheres other agents to aid in the distribution and/or delivery of the biodegradable microparticles to targeted sites and/or targeted cells. Such carriers are well known to those of ordinary skill in the art.

Preferably, said pharmaceutical composition does not contain a high amount of microparticles which are not according to the invention. For instance, if the pharmaceutical composition contains too many microparticles which are too big, it is not very effective. Therefore, at least 50, 60, 70, 80, 90 percent of the microparticles present in the pharmaceutical composition are the biodegradable microparticles according to the invention.

In a preferred embodiment, the biodegradable microparticles according to the invention are formulated into pharmaceutical compositions that can be made into dosage forms, in particular oral solid dosage forms such as capsules and compressed tablets, as are well known in the art.

Compressed tablets are formulated from pharmaceutical compositions containing the biodegradable microparticles, or using pharmaceutical carrier particles bearing such microparticles, and pharmacologically inert (pharmaceutically acceptable) additives or excipients.

For making a tablet, it is typically desirable to include one or more benign pharmaceutical excipients in the pharmaceutical composition. The pharmaceutical composition of the present invention may contain one or more diluents added to make the tablet larger and, hence, easier for the patient and caregiver to handle. Common diluents are microcrystalline cellulose (e.g. Avicel®), microfine cellulose, lactose, starch, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit®), potassium chloride, powdered cellulose, sodium chloride, sorbitol and talc.

Binders also may be included in tablet formulations to help hold the tablet together after compression. Some typical binders are acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel®), hydroxypropyl methyl cellulose (e.g. Methocel®), liquid glucose, magnesium aluminum silicate, maltodextrin, methylcellulose, polymethacrylates, povidone (e.g. Kollidon®, Plasdone®), pregelatinized starch, sodium alginate and starch.

The tablet may further include a disintegrant to accelerate disintegration of the tablet in the patient's stomach. Disintegrants include alginic acid, carboxymethyl cellulose calcium, carboxymethylcellulose sodium, colloidal silicon dioxide, croscarmellose sodium (e.g. Ac-Di-Sol®, Primellose®), crospovidone (e.g. Kollidon®, Polyplasdone®), guar gum, magnesium aluminum silicate, methyl cellulose, microcrystalline cellulose, polacrilin potassium, powdered cellulose, pregelatinized starch, sodium alginate, sodium starch glycolate (e.g. Explotab®) and starch.

A pharmaceutical composition for making compressed tablets may further include glidants, lubricants, flavorings, colorants and other commonly used excipients.

Liquid oral pharmaceutical compositions of the present invention comprise biodegradable microparticles according to the invention and a liquid carrier such as water, vegetable oil, alcohol, polyethylene glycol, propylene glycol or glycerin, most preferably water.

Liquid oral pharmaceutical compositions may contain emulsifying agents to disperse uniformly throughout the composition the active ingredient, drug delivery vehicle, or excipient having low solubility in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol and cetyl alcohol.

Liquid oral pharmaceutical compositions of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth and xanthan gum.

The liquid oral pharmaceutical composition also may contain sweetening agents, such as sorbitol, saccharin, sodium saccharin, sucrose, aspartame, fructose, mannitol and invert sugar; preservatives and chelating agents such as alcohol, sodium benzoate, butylated hydroxy toluene, butylated hydroxyanisole and ethylenediamine tetraacetic acid; and buffers such as guconic acid, lactic acid, citric acid or acetic acid, sodium gluconate, sodium lactate, sodium citrate or sodium acetate.

In other preferred embodiments, the pharmaceutical composition according to the invention is a composition suitable for topical application. Suitable pharmaceutical compositions for topical application of an active proteinaceous compound are well known in the art. Suitable topical pharmaceutical compositions may include one or more drying agents, preferably zinc-oxide, a solvent, preferably a monohydric alkanol, a humectant, preferably glycerol and/or a viscosity-building agent, preferably bentonite, mixed with water to form a lotion or cream.

The invention will now be illustrated by way of the following, nonlimiting Examples.

### EXAMPLES

### Example 1. Activation of an immune-regulatory macrophage response by microsphere-encapsulated CRYAB in the presence of human serum

### Method

### Synthesis of hydrophilic polyester: copolymers of 3S-(benzyloxymethyl)-6S-methyl-1, 4-dioxane-2, 5-dione with L-lactide (BHMG) with D,L-lactide

3S-(benzyloxymethyl)-6S-methyl-1, 4-dioxane-2, 5-dione (BMMG) was synthesized according to Leemhuis et al. [Macromolecules 39: 3500-3508 (2006)], Copolymers of BMMG and D,L-lactide (monomer ratio 35/65 and 50/50 % mol/mol) were synthesized by melt copolymerization as described by Ghassemi et al. [J. Control. Release 138: 57-63 (2009)]. After copolymerization, the protecting benzyl groups were removed, and the composition of the poly(lactic-co-hydroxymethyl glycolic acid) (PLHMGA) copolymers was established ¹H-NMR. The appropriate glass-transition temperature of the copolymers was verified by differential scanning calorimetry, and their expected molecular weights by size exclusion chromatography. ¹H-NMR analyses of the copolymers (both benzyl protected and de-protected) confirmed that the copolymer compositions matched those of the feed ratio of the monomers, and that deprotection was complete. Yield of the copolymers was typically between 90 and 100%.

### Preparation and characterization of CRYAB-loaded PLHMGA & PLGA microparticles

CRYAB-loaded microspheres of PLHMGA polymers (Fig. 1), or the more widely used poly(lactic-co- glycolic acid) (PLGA) polymers, were prepared by a double-emulsion solvent-evaporation technique, as described by Wang et al. [Journal of Controlled Release 82: 289-3073 (2002)]. Briefly, 300 µl of a 12.5 mg/ml CRYAB solution in phosphate-buffered saline was added drop-wise to 3 ml of a solution of 10 % (w/v) PLHMGA or PLGA solution in dichloromethane (DCM). The water/DCM two-phase system was emulsified by using an IKA homogenizer (IKA Werke Labortechnik, Staufen, Germany) for 1 min at 35,000 rpm. Subsequently, the resulting water-in-oil (w/o) emulsion was added to 30 ml of 5% (w/v) polyvinyl alcohol (PVA) containing 150 mM NaCl, pH 7.4 and the mixture was emulsified for 2 min by using an IKA homogenizer at 35,000 rpm. The resulting water-in oil-in water (w/o/w) emulsion was transferred to a round-bottom flask, and DCM was evaporated under vacuum at room temperature. Next, the microspheres were collected by centrifugation at 25,000 g for 20 min, washed three times with 50 ml water, lyophilized overnight and stored dry at 4°C until used for experiments. Particles were characterized for size by dynamic light scattering and accusizer analysis, and examined for morphology by scanning electron microscopy.

The results indicated the formation of spherical particles with a size distribution of 0.5-3.5 µm for PLGA microspheres, while the PLHMGA particles had a narrower particle size distribution in the range of 0.2-2 µm. Scanning electron micrographs of the microparticles thus obtained are presented in Fig. 2.

### Example 2. Activation of human macrophages with free CRYAB or microsphere-encapsulated CRYAB.

Human peripheral blood mononuclear cells were isolated from whole blood obtained from healthy donors. CD14⁺ monocytes were isolated by positive selection using magnetic beads coated with an antibody against CD14. Such purified monocytes were cultured for seven days in standard culture medium containing 10% human serum in the presence of macrophage-colony-stimulating factor (M-CSF) to induce their differentiation into macrophages. After seven days, when cells were fully differentiated, half the culture medium was removed, and replaced with an equal volume of fresh medium with 10% human serum, and containing various stimuli at different concentrations. Stimuli included free soluble human recombinant CRYAB, and preparations of CRYAB-loaded PLHMGA or PLGA microspheres, prepared in the same culture medium. Empty microspheres, containing no protein, were used as controls. After 18-20 h of culture, when macrophages had visibly phagocytosed the majority of microparticles, the culture plates were centrifuged at 1,250 g for 20 min at 4°C. Finally, all culture medium supernatants were individually collected for quantification of IL-10 using a commercial ELISA kit.

### Example 3. Microspheres containing CRYAB, but not empty microspheres, induce IL-10 production by human macrophages (cf Fig. 6).

Human peripheral blood mononuclear cells were isolated from whole blood obtained from healthy donors. CD14⁺ monocytes were isolated by positive selection using magnetic beads coated with an antibody against CD 14. Such purified monocytes were cultured for seven days in the presence of macrophage-colony-stimulating factor (M-CSF) to induce their differentiation into macrophages. After seven days, when cells were fully differentiated, half the culture medium was removed, and replaced with an equal volume of fresh medium containing various stimuli at different concentrations. Stimuli included varying concentrations of CRYAB-loaded PLGA microspheres, or mixtures of CRYAB-loaded and empty PLGA microspheres of the same dimensions, in concentrations indicated in Fig. 6. After 18-20 h of culture, medium supernatants were individually examined for IL-10 as described above.

### Example 4. Inhibition of an antigen-specific proliferative response by human peripheral blood T cells by microsphere-encapsulated CRYAB but not by free soluble CRYAB (cf Fig. 8).

Human peripheral blood mononuclear cells (PBMC) were isolated from whole blood buffy coats obtained from healthy donors. Such purified PBMC were first labelled with the fluorescent dye carboxylfluorescein succinimidyl ester (CFSE). This dye, which stably labels intracellular proteins, is commonly used for T-cell proliferation assays, since such proliferation can be visualized and quantified by the stepwise dilution of the intracellular fluorescent label as the consequence of cell division during proliferation. CFSE-labelled PBMC were cultured for nine days in the presence of either 200 µg/mL human recombinant CRYAB or 0.2 µg/mL tetanus toxoid, two test antigens to which most humans have established memory T-cell responses. At the day of culture, PBMC with or without test antigens were additionally supplied with increasing concentrations of either free CRYAB up to 30 µg/mL or PLGA-microsphere-encapsulated CRYAB up to 30 µg/mL total microsphere mass (1% of which is CRYAB protein). After nine days in culture, PBMC were harvested and stained with fluorescently labelled antibodies for the surface markers CD4 (helper T-cel marker) and CD45RO (memory T-cell marker) and subjected to analysis by flowcytometry. The intensity of the fluorescently labelled markers CD4 or CD45RO allows flowcytometric analysis to focus on either helper T cells or memory T cells within the population of PBMC, while the intensity of the fluorescent CFSE label allows the identification and quantification of the fraction of such T cells that have proliferated during culture, since these are characterized by a diluted, and therefore dimmed CFSE signal. Shown in Fig. 8 are mean ± standard deviations. Statistical significance was calculated using an ANOVA test.

### Example 5. Suppression of cigarette-smoke-induced lung inflammation by CRYAB-containing microspheres, but not by free soluble CRYAB, even at much higher doses (cf Fig. 9).

For a period of five days, mice (groups n=6 or n=7) were exposed to cigarette smoke twice a day for 30 min. As a treatment, free soluble CRYAB in PBS, CRYAB-containing PLGA microspheres resuspended in PBS, or PBS alone were administered intratracheally twice a day under light isofluorane anaesthesia. On day 6, animals were sacrificed and broncho-alveolar lavages were collected from all animals, and individually examined for numbers of macrophages, eosinophils, neutrophils, and lymphocytes. All experiments were performed by qualified personnel, with prior written approval from an animal experimentation's ethical committee, in accordance with all local regulations and legal stipulations. Shown in Figs. 9A and B are mean ± standard error of the mean. Statistical significance was calculated using unpaired Student's t-tests.

## Claims

1. A biodegradable microparticle for use in the treatment of inflammatory disease, wherein the microparticle has a diameter between 0.2 and 3.5 micrometer and comprises a pharmaceutically effective amount of at least one small heat-shock protein that induces IL-10 production in macrophages, wherein said small heat-shock protein comprises an amino acid sequence identity of at least 50% to any of the sequences listed as SEQ ID NOs:1 and 12-26.

2. Biodegradable microparticle for use according to claim 1, wherein said inflammatory disease is an acute or chronic inflammatory disorder of the skin, mucosa, the lungs, the nervous system the vascular system, the pancreas or of a joint, preferably selected from dermatitis, psoriasis, eczema, Crohn's disease, ulcerative colitis, paradontitis, lichen planus, lichen sclerosus, chronic obstructive pulmonary disorder, emphysema, Alzheimer disease, Parkinson disease, dementia, optic neuritis, encephalitis, inflammatory peripheral neuropathies, atherosclerosis, vasculitis, rheumatoid arthritis or diabetes,

3. Biodegradable microparticle for use according to claim 1 or 2, wherein said at least one small heat-shock protein is the protein with the amino acid sequence selected from the group of SEQ ID NOs: 2-11, preferably SEQ ID NO: 2.

4. Biodegradable microparticle for use according to any one of claims 1-3, wherein said biodegradable microparticle is biocompatible.

5. Biodegradable microparticle for use according to any one of claims 1-4, wherein said microparticle comprises a (co)polymer of lactic acid and/or glycolic acid, preferably selected from caprolactone, polylactic acid (PLA), polylactic-co-glycolic acid (PLGA) or polylactic-co-hydroxymethylglycolic acid (PLHMGA),

6. Biodegradable microparticle for use according to any one of claims 1-5, wherein said biodegradable microparticle has a maximal diameter between 1 and 2 micrometer.

7. Pharmaceutical composition for use in the treatment of inflammatory disease comprising an effective dose of the biodegradable microparticle for use according to claim any one of claims 1-6,
preferably wherein at least 50, 60, 70, 80, or 90 percent of the microparticles present in the pharmaceutical composition are biodegradable microparticles for use according to any one of claims 1-6.

8. Biodegradable microparticles having a mean diameter between 0.2 and 3.5 micrometer and comprising a pharmaceutically effective amount of at least one small heat-shock protein that induces IL-10 production in macrophages, said small heat-shock protein comprising an amino acid sequence identity of at least 50% to any of the sequences listed as SEQ ID NOs:1 and 12-26, preferably wherein the microparticles are suitable for being phagocytosed by phagocytes, preferably wherein the microparticles are suitable to activate phagocytes.

9. Biodegradable microparticles according to claim 8, wherein said at least one small heat-shock protein is the protein with the amino acid sequence selected from the group of SEQ ID NOs: 2-11, preferably SEQ ID NO: 2,
preferably wherein the biodegradable microparticles are biocompatible, preferably wherein said microparticles comprise a (co)polymer of lactic acid and/or glycolic acid, preferably selected from caprolactone, polylactic acid (PLA), polylactic-co-glycolic acid (PLGA) or polylactic-co-hydroxymethylglycolic acid (PLHMGA),
and preferably wherein said biodegradable microparticle has a maximal diameter between 1 and 2 micrometer.

10. Composition comprising a biodegradable microparticle according to claim 8 or 9, wherein said biodegradable microparticle has a diameter between 0.2 and 3.5 micrometer and comprises a pharmaceutically effective amount of at least one small heat-shock protein that induces IL-10 production in macrophages, wherein said small heat-shock protein comprises an amino acid sequence identity of at least 50% to any of the sequences listed as SEQ ID NOs:1 and 12-26, wherein at least 50 percent of the microparticles present in the composition are said biodegradable microparticle,
preferably wherein said at least one small heat-shock protein is the protein with the amino acid sequence selected from the group of SEQ ID NOs: 2-11, preferably SEQ ID NO: 2,
preferably wherein said biodegradable microparticle is biocompatible, preferably wherein said microparticle comprises a (co)polymer of lactic acid and/or glycolic acid, preferably selected from caprolactone, polylactic acid (PLA), polylactic-co-glycolic acid (PLGA) or polylactic-co-hydroxymethylglycolic acid (PLHMGA),
preferably wherein said biodegradable microparticle has a maximal diameter between 1 and 2 micrometer,
and preferably wherein the composition is a pharmaceutical composition comprising an effective dose of said biodegrable microparticle..

11. Biodegradable microparticles according to claim 8 or 9, or a composition according to claim 10 for use in a treatment of a disease, preferably for use in a treatment of an inflammatory disease, preferably wherein said inflammatory disease is an acute or chronic inflammatory disorder of the skin, mucosa, the lungs, the nervous system the vascular system, the pancreas or of a joint, preferably dermatitis, psoriasis, eczema, Crohn's disease, ulcerative colitis, paradontitis, lichen planus, lichen sclerosus, chronic obstructive pulmonary disorder, emphysema, Alzheimer disease, Parkinson disease, dementia, optic neuritis, encephalitis, inflammatory peripheral neuropathies, atherosclerosis, vasculitis, rheumatoid arthritis or diabetes.

12. Pharmaceutical composition comprising an effective dose of the microparticles according to any one of claims 8-9.

13. Pharmaceutical composition according to claim 12, wherein at least 50, 60, 70, 80, 90 percent of the microparticles present in the pharmaceutical composition are biodegradable microparticles according to any one of claims 8-9.

14. Method for producing a biodegradable microparticle as defined in any one of claims 1-6, 8-9 or a composition according to claim 7, 10 or 12-13 comprising steps of:
a. mixing an aqueous solution comprising a small heat-shock protein as defined in claim 1 or 3 with a solution of caprolactone, PLA, PLGA or PLHMGA in volatile organic solvent to provide a water/ volatile organic solvent two phase system;
b. emulsifying said water/ volatile organic solvent two phase system to provide a water-in-oil emulsion;
c. adding the water-in-oil emulsion from step b to an aqueous solution comprising polyvinyl alcohol and emulsifying the resulting mixture to provide a water-in-oil-in-water emulsion;
d. allow the volatile organic solvent to evaporate from said water-in-oil-in-water emulsion and allow the formation of biodegradable microparticles during said evaporation.

## Patentansprüche

1. Biologisch abbaubares Mikropartikel zur Verwendung bei der Behandlung entzündlicher Krankheiten, wobei das Mikropartikel einen Durchmesser zwischen 0,2 und 3,5 Mikrometer hat und eine pharmazeutisch wirksame Menge von mindestens einem kleinen Hitzeschockprotein, das IL-10 Produktion in Makrophagen induziert, umfasst, wobei das kleine Hitzeschockprotein eine Aminosäure-Sequenzidentität von mindestens 50 % zu jeder beliebigen der Sequenzen, aufgelistet als SEQ ID Nummern:1 und 12-26, umfasst.

2. Biologisch abbaubares Mikropartikel zur Verwendung nach Anspruch 1, wobei die entzündliche Erkrankung eine akute oder chronische entzündliche Störung der Haut, der Schleimhaut, der Lungen, des Nervensystems, des Gefäßsystems, der Bauchspeicheldrüse oder eines Gelenks ist, bevorzugt ausgewählt aus Dermatitis, Psoriasis, Ekzem, Morbus Crohn, eiternder Colitis, Parodontitis, Liehen planus, Liehen sclerosus, chronisch obstruktiver Lungenerkrankung, Emphysem, Alzheimer, Parkinson, Demenz, optischer Neuritis, Enzephalitis, entzündlichen peripheren Neuropathien, Atherosklerose, Gefäßentzündung, rheumatoider Arthritis oder Diabetes.

3. Biologisch abbaubares Mikropartikel zur Verwendung nach Anspruch 1 oder 2, wobei das mindestens eine kleine Hitzeschockprotein das Protein ist mit der Aminosäuresequenz, ausgewählt aus der Gruppe von SEQ ID Nummern: 2-11, bevorzugt SEQ ID Nr.:2.

4. Biologisch abbaubares Mikropartikel zur Verwendung nach einem der Ansprüche 1-3, wobei das biologisch abbaubare Mikropartikel biokompatibel ist.

5. Biologisch abbaubares Mikropartikel zur Verwendung nach einem der Ansprüche 1-4, wobei das Mikropartikel ein (Co)polymer von Milchsäure und/oder Glycolsäure umfasst, bevorzugt ausgewählt aus Caprolacton, Polymilchsäure (PLA), Polymilch-co-glycolsäure (PLGA) oder Polymilch-cohydroxymethylglycolsäure (PLHMGA).

6. Biologisch abbaubares Mikropartikel zur Verwendung nach einem der Ansprüche 1-5, wobei das biologisch abbaubare Mikropartikel einen maximalen Durchmesser zwischen 1 und 2 Mikrometer hat.

7. Pharmazeutische Zusammensetzung zur Verwendung bei der Behandlung einer entzündlichen Krankheit, umfassend eine wirksame Dosis des biologisch abbaubaren Mikropartikels zur Verwendung nach einem der Ansprüche 1-6, bevorzugt wobei mindestens 50, 60, 70, 80 oder 90 Prozent der in der pharmazeutischen Zusammensetzung anwesenden Mikropartikel biologisch abbaubare Mikropartikel zur Verwendung nach einem der Ansprüche 1-6 sind.

8. Biologisch abbaubare Mikropartikel mit einem mittleren Durchmesser zwischen 0,2 und 3,5 Mikrometer und umfassend eine pharmazeutisch wirksame Menge von mindestens einem kleinen Hitzeschockprotein, das die IL-10 Produktion in Makrophagen induziert, wobei das kleine Hitzeschockprotein eine Aminosäure-Sequenzidentität von mindestens 50 % zu einer beliebigen der Sequenzen, aufgelistet als SEQ ID Nummern:1 und 12-26 umfasst, bevorzugt wobei die Mikropartikel geeignet sind, durch Phagozyten phagozytiert zu werden, bevorzugt wobei die Mikropartikel geeignet sind, Phagozyten zu aktivieren.

9. Biologisch abbaubare Mikropartikel nach Anspruch 8, wobei das mindestens eine kleine Hitzeschockprotein das Protein ist mit der Aminosäuresequenz, ausgewählt aus der Gruppe von SEQ ID Nummern: 2-11, bevorzugt SEQ ID Nr.:2,
bevorzugt wobei die biologisch abbaubaren Mikropartikel biokompatibel sind,
bevorzugt wobei die Mikropartikel ein (Co)polymer von Milchsäure und/oder Glycolsäure umfassen, bevorzugt ausgewählt aus Caprolacton, Polymilchsäure (PLA), Polymilch-co-glycolsäure (PLGA) oder Polymilch-cohydroxymethylglycolsäure (PLHMGA), und
bevorzugt wobei das biologisch abbaubare Mikropartikel einen maximalen Durchmesser zwischen 1 und 2 Mikrometer hat.

10. Zusammensetzung, umfassend ein biologisch abbaubares Mikropartikel nach Anspruch 8 oder 9, wobei das biologisch abbaubare Mikropartikel einen Durchmesser zwischen 0,2 und 3,5 Mikrometer hat und eine pharmazeutisch wirksame Menge von mindestens einem kleinen Hitzeschockprotein, das IL-10 Produktion in Makrophagen induziert, umfasst, wobei das kleine Hitzeschockprotein eine Aminosäure-Sequenzidentität von mindestens 50 % zu jeder beliebigen der Sequenzen, aufgelistet als SEQ ID Nummern:1 und 12-26, umfasst, wobei mindestens 50 Prozent der in der Zusammensetzung anwesenden Mikropartikel das biologisch abbaubare Mikropartikel ist,
bevorzugt wobei das mindestens eine kleine Hitzeschockprotein das Protein mit der Aminosäuresequenz, ausgewählt aus der Gruppe von SEQ ID Nummern: 2-11, bevorzugt SEQ ID Nr.: 2, ist,
bevorzugt wobei das biologisch abbaubare Mikropartikel biokompatibel ist,
bevorzugt wobei das Mikropartikel ein (Co)polymer von Milchsäure und/oder Glycolsäure umfasst, bevorzugt ausgewählt aus Caprolacton, Polymilchsäure (PLA), Polymilch-co-glycolsäure (PLGA) oder Polymilch-cohydroxymethylglycolsäure (PLHMGA),
bevorzugt wobei das biologisch abbaubare Mikropartikel einen maximalen Durchmesser zwischen 1 und 2 Mikrometer hat,
und bevorzugt wobei die Zusammensetzung eine pharmazeutische Zusammensetzung umfassend eine wirksame Dosis des biologisch abbaubaren Mikropartikels ist.

11. Biologisch abbaubare Mikropartikel nach Anspruch 8 oder 9 oder eine Zusammensetzung nach Anspruch 10 zur Verwendung bei einer Behandlung einer Krankheit, bevorzugt zur Verwendung bei einer Behandlung einer entzündlichen Erkrankung, bevorzugt wobei die entzündliche Erkrankung eine akute oder chronische entzündliche Störung der Haut, der Schleimhaut, der Lungen, des Nervensystems, des Gefäßsystems, der Bauchspeicheldrüse oder eines Gelenks ist, bevorzugt Dermatitis, Psoriasis, Ekzem, Morbus Crohn, eiternde Colitis, Parodontitis, Liehen planus, Liehen sclerosus, chronisch obstruktive Lungenerkrankung, Emphysem, Alzheimer, Parkinson, Demenz, optische Neuritis, Enzephalitis, entzündliche periphere Neuropathien, Atherosklerose, Gefäßentzündung, rheumatoide Arthritis oder Diabetes ist.

12. Pharmazeutische Zusammensetzung umfassend eine wirksame Dosis der Mikropartikel nach einem der Ansprüche 8-9.

13. Pharmazeutische Zusammensetzung nach Anspruch 12, wobei mindestens 50, 60, 70, 80, 90 Prozent der in der pharmazeutischen Zusammensetzung anwesenden Mikropartikel biologisch abbaubare Mikropartikel nach einem der Ansprüche 8-9 sind.

14. Verfahren zur Herstellung eines biologisch abbaubaren Mikropartikels, wie in einem der Ansprüche 1-6, 8-9, oder einer Zusammensetzung nach Anspruch 7, 10 oder 12-13 definiert, umfassend folgende Schritte, in denen:
a. eine wässrigen Lösung, umfassend ein kleines Hitzeschockprotein wie in Anspruch 1 oder 3 definiert mit einer Lösung aus Caprolacton, PLA, PLGA oder PLHMGA in einem flüchtigen organischen Lösungsmittel gemischt wird, um ein Zweiphasensystem aus Wasser/flüchtigem organischen Lösungsmittel bereitzustellen;
b. das Zweiphasensystem aus Wasser/flüchtigem organischen Lösungsmittel emulgiert wird, um eine Wasser-in-Öl-Emulsion bereitzustellen;
c. die Wasser-in-Öl-Emulsion aus Schritt b zu einer wässrigen Lösung, umfassend Polyvinylalkohol, hinzugefügt wird und das erhaltene Gemisch emulgiert wird, um eine Wasser-in-Öl-in-Wasser-Emulsion bereitzustellen;
d. es dem flüchtigen organischen Lösungsmittel ermöglicht wird, aus der Wasser-in-Öl-in-Wasser-Emulsion zu verdunsten, und während der Verdunstung die Bildung biologisch abbaubarer Mikropartikel ermöglicht wird.

## Revendications

1. Une microparticule biodégradable pour une utilisation dans le traitement de maladies inflammatoires, laquelle microparticule présente un diamètre compris entre 0,2 et 3,5 micromètres et comprend une quantité pharmaceutiquement efficace d'au moins une petite protéine de choc thermique qui induit la production de IL-10 dans les macrophages, ladite petite protéine de choc thermique comprenant une identité de séquence d'acides aminés d'au moins 50% vis-à-vis de n'importe laquelle des séquences de la liste formée par SEQ ID NO: 1 et 12 à 26.

2. Microparticule biodégradable pour une utilisation selon la revendication 1, dans laquelle ladite maladie inflammatoire est une maladie inflammatoire aiguée ou chronique de la peau, des muqueuses, des poumons, du système nerveux, du système vasculaire, du pancréas ou d'une articulation, de préférence choisie parmi la dermatite, du psoriasis, de l'eczéma, de la maladie de Crohn, de la colite ulcéreuse, de la parodontite, du lichen plan, du lichen scléro-atrophique, de la maladie pulmonaire obstructive chronique, de l'emphysème, de la maladie d'Alzheimer, de la maladie de Parkinson, de la démence, de la névrite optique, de l'encéphalite, des neuropathies périphériques inflammatoires, de l'athérosclérose, de la vasculite, de l'arthrite rhumatoïde ou des diabètes.

3. Microparticule biodégradable pour une utilisation selon la revendication 1 ou 2, dans laquelle ladite au moins une petite protéine de choc thermique présente la séquence d'acides aminés choisie dans le groupe des SEQ ID NO: 2 à 11, de préférence SEQ ID NO: 2.

4. Microparticule biodégradable pour une utilisation selon l'une quelconque des revendications 1 à 3, dans laquelle ladite microparticule biodégradable est biocompatible.

5. Microparticule biodégradable pour une utilisation selon l'une quelconque des revendications 1 à 4, laquelle microparticule comprend un (co)polymère d'acide lactique et/ou d'acide glycolique, de préférence choisi parmi le caprolactone, l'acide polylactique (PLA), l'acide polylactique-co-glycolique (PLGA) ou l'acide polylactique-co-hydroxyméthylglycolique (PLHMGA).

6. Microparticule biodégradable pour une utilisation selon l'une quelconque des revendications 1 à 5, laquelle microparticule biodégradable présente un diamètre maximal compris entre 1 et 2 micromètres.

7. Composition pharmaceutique pour une utilisation dans le traitement de maladies inflammatoires comprenant une dose efficace de microparticule biodégradable pour une utilisation selon l'une quelconque des revendications 1 à 6, de préférence où au moins 50, 60, 70, 80, 90 pour cent des microparticules présentes dans la composition pharmaceutique sont des microparticules biodégradables pour une utilisation selon l'une quelconque des revendications 1 à 6.

8. Microparticules biodégradables présentant un diamètre moyen compris entre 0,2 et 3,5 micromètres et comprenant une quantité pharmaceutiquement efficace d'au moins une petite protéine de choc thermique qui induit la production de IL-10 dans les macrophages, ladite petite protéine de choc thermique comprenant une identité de séquences d'acides aminés d'au moins 50% vis-à-vis de n'importe laquelle des séquences de la liste formée par SEQ ID NO: 1 et 12 à 26, de préférence lesdites microparticules étant susceptibles d'être phagocytées par des phagocytes, de préférence lesdites microparticules étant susceptibles d'activer des phagocytes.

9. Microparticules biodégradables selon la revendication 8, dans lesquelles ladite au moins une petite protéine de choc thermique est la protéine dont la séquence d'acide aminé est choisie dans le groupe de séquences SEQ ID NOs: 2-11, de préférence SEQ ID NO: 2,
de préférence où les microparticules biodégradables sont biocompatibles,
de préférence où ces microparticules comprennent un (co)polymère d'acide lactique et/ou d'acide glycolique, de préférence choisi parmi le caprolactone, l'acide polylactique (PLA), l'acide polylactique-co-glycolique (PLGA) ou l'acide polylactique-co-hydroxyméthyl-glycolique (PLHMGA),
et de préférence où ces microparticules biodégradables présentent un diamètre maximum compris entre 1 et 2 micromètres.

10. Composition comprenant une microparticule biodégradable selon la revendication 8 ou 9, dans laquelle ladite microparticule biodégradable présente un diamètre compris entre 0,2 et 3,5 micromètres et comprend une quantité pharmaceutiquement efficace d'au moins une petite protéine de choc thermique qui induit la production de IL-10 dans les macrophages, dans laquelle ladite petite protéine de choc thermique comprend une identité de séquences d'acides aminés d'au moins 50% vis-à-vis de n'importe laquelle des séquences de la liste formée par SEQ ID NO: 1 et 12 à 26, dans laquelle au moins solo des microparticules présentes dans la composition consistent en cette microparticule biodégradable, de préférence dans laquelle au moins une petite protéine de choc thermique est la protéine dont la séquence d'acide aminé est choisie dans le groupe de séquences SEQ ID NOs: 2-11, de préférence SEQ ID NO: 2,
de préférence dans laquelle les microparticules biodégradables sont biocompatibles,
de préférence dans laquelle cette microparticule comprend un (co)polymère d'acide lactique et/ou d'acide glycolique, de préférence choisi parmi le caprolactone, l'acide polylactique (PLA), l'acide polylactique-co-glycolique (PLGA) ou l'acide polylactique-co-hydroxyméthylglycolique (PLHMGA),
de préférence dans laquelle ces microparticule s biodégradables présentent un diamètre maximum compris entre 1 et 2 micromètres
et de préférence où la composition est une composition pharmaceutique comprenant une dose efficace de la dite microparticule biodégradable.

11. Microparticules biodégradables selon la revendication 8 ou 9, ou une composition selon la revendication 10, pour une utilisation dans le traitement d'une maladie, de préférence pour une utilisation dans le traitement d'une maladie inflammatoire, de préférence où ladite maladie inflammatoire est une maladie inflammatoire aiguée ou chronique de la peau, des muqueuses, des poumons, du système nerveux, du système vasculaire, du pancréas ou d'une articulation, de préférence de la dermatite, du psoriasis, de l'eczéma, de la maladie de Crohn, de la colite ulcéreuse, de la parodontite, du lichen plan, du lichen scléro-atrophique, de la maladie pulmonaire obstructive chronique, de l'emphysème, de la maladie d'Alzheimer, de la maladie de Parkinson, de la démence, de la névrite optique, de l'encéphalite, des neuropathies périphériques inflammatoires, de l'athérosclérose, de la vasculite, de l'arthrite rhumatoïde ou des diabètes.

12. Composition pharmaceutique comprenant une dose efficace de microparticules selon une quelconque des revendications 8 ou 9.

13. Composition pharmaceutique selon la revendication 12, dans laquelle au moins 50, 60, 70, 80, 90 pour cent des microparticules présentes dans la composition pharmaceutique sont des microparticules biodégradables selon l'une quelconque des revendications 8 ou 9.

14. Procédé de fabrication d'une microparticule biodégradable tel que défini selon l'une quelconque des revendications 1 à 6, 8-9 ou une composition selon les revendications 7, 10 ou 12-13, comprenant les étapes consistant à:
a. mélanger une solution aqueuse comprenant une petite protéine de choc thermique telle que définie dans l'une des revendications 1 ou 3, avec une solution de caprolactone, PLA, PLGA PLHMGA dans un solvant organique volatil pour former un système biphasique eau/solvant organique volatil;
b. émulsifier ledit système biphasique solvant eau/solvant organique volatil pour obtenir une émulsion eau-dans-huile;
c. additionner l'émulsion eau-dans-huile de l'étape b à une solution aqueuse comprenant de l'alcool polyvinylique et émulsifier le mélange obtenu pour obtenir une émulsion eau-dans-huile-dans-eau;
d. laisser s'évaporer le solvant organique volatil de ladite émulsion eau-dans-huile-dans-eau, et permettre la formation de microparticules biodégradables durant ladite évaporation.


### SEQUENCE LISTING

<110> Delta Crystallon B.V.
<120> Microparticles comprising a small heat-shock protein
<130> P91820PC00
<151> EP 10169869.4
<160> 26
<170> PatentIn version 3.3
<210> 1
   <211> 81
   <212> PRT
   <213> Homo sapiens
<220>
   <221> MISC-FEATURE
   <222> (1)..(81)
   <223> α-Crystallin domain of HSPB5
<400> 1
   Met Arg Leu Glu Lys Asp Arg Phe Ser Val Asn Leu Asp Val Lys His
   1               5                   10                  15
   Phe Ser Pro Glu Glu Leu Lys Val Lys Val Leu Gly Asp Val Ile Glu
               20                  25                  30
   Val His Gly Lys His Glu Glu Arg Gln Asp Glu His Gly Phe Ile Ser
           35                  40                  45
   Arg Glu Phe His Arg Lys Tyr Arg Ile Pro Ala Asp Val Asp Pro Leu
       50                  55                  60
   Thr Ile Thr Ser Ser Leu Ser Ser Asp Gly Val Leu Thr Val Asn Gly
   65                  70                  75                  80
   Pro
<210> 2
   <211> 175
   <212> PRT
   <213> Homo sapiens
<220>
   <221> MISC-FEATURE
   <222> (1)..(175)
   <223> HSPB5
<220>
   <221> DOMAIN
   <222> (68)..(148)
   <223> α-Crystallin domain
<400> 2
<210> 3
   <211> 173
   <212> PRT
   <213> Homo sapiens
<220>
   <221> MISC-FEATURE
   <222> (1)..(173)
   <223> HSPB4
<220>
   <221> DOMAIN
   <222> (64)..(144)
   <223> α-Crystallin domain
<400> 3
   Met Asp Val Thr Ile Gln His Pro Trp Phe Lys Arg Thr Leu Gly Pro
   1 5 10 15
   Phe Tyr Pro Ser Arg Leu Phe Asp Gln Phe Phe Gly Glu Gly Leu Phe
   20 25 30
   Glu Tyr Asp Leu Leu Pro Phe Leu Ser Ser Thr Ile Ser Pro Tyr Tyr
   35 40 45
   Arg Gln Ser Leu Phe Arg Thr Val Leu Asp Ser Gly Ile Ser Glu Val
   50 55 60
   Arg Ser Asp Arg Asp Lys Phe Val Ile Phe Leu Asp Val Lys His Phe
   65 70 75 80
   Ser Pro Glu Asp Leu Thr Val Lys Val Gln Asp Asp Phe Val Glu Ile
   85 90 95
   His Gly Lys His Asn Glu Arg Gln Asp Asp His Gly Tyr Ile Ser Arg
   100 105 110
   Glu Phe His Arg Arg Tyr Arg Leu Pro Ser Asn Val Asp Gln Ser Ala
   115 120 125
   Leu Ser Cys Ser Leu Ser Ala Asp Gly Met Leu Thr Phe Cys Gly Pro
   130 135 140
   Lys Ile Gln Thr Gly Leu Asp Ala Thr His Ala Glu Arg Ala Ile Pro
   145 150 155 160
   Val Ser Arg Glu Glu Lys Pro Thr Ser Ala Pro Ser Ser
   165 170
<210> 4
   <211> 205
   <212> PRT
   <213> Homo sapiens
<220>
   <221> MISC-FEATURE
   <222> (1)..(205)
   <223> HSPB1
<220>
   <221> DOMAIN
   <222> (88)..(168)
   <223> α-Crystallin domain
<400> 4
<210> 5
   <211> 182
   <212> PRT
   <213> Homo sapiens
<220>
   <221> MISC-FEATURE
   <222> (1)..(182)
   <223> HSPB2
<220>
   <221> DOMAIN
   <222> (67)..(147)
   <223> α-Crystallin domain
<400> 5
   Met Ser Gly Arg Ser Val Pro His Ala His Pro Ala Thr Ala Glu Tyr
   1               5                   10                  15
   Glu Phe Ala Asn Pro Ser Arg Leu Gly Glu Gln Arg Phe Gly Glu Gly
               20                  25                  30
   Leu Leu Pro Glu Glu Ile Leu Thr Pro Thr Leu Tyr His Gly Tyr Tyr
           35                  40                  45
   Val Arg Pro Arg Ala Ala Pro Ala Gly Glu Gly Ser Arg Ala Gly Ala
       50                  55                  60
   Ser Glu Leu Arg Leu Ser Glu Gly Lys Phe Gln Ala Phe Leu Asp Val
   65                  70                  75                  80
   Ser His Phe Thr Pro Asp Glu Val Thr Val Arg Thr Val Asp Asn Leu
                   85                  90                  95
   Leu Glu Val Ser Ala Arg His Pro Gln Arg Leu Asp Arg His Gly Phe
               100                 105                 110
   Val Ser Arg Glu Phe Cys Arg Thr Tyr Val Leu Pro Ala Asp Val Asp
           115                 120                 125
   Pro Trp Arg Val Arg Ala Ala Leu Ser His Asp Gly Ile Leu Asn Leu
       130                 135                 140
   Glu Ala Pro Arg Gly Gly Arg His Leu Asp Thr Glu Val Asn Glu Val
   145                 150                 155                 160
   Tyr Ile Ser Leu Leu Pro Ala Pro Pro Asp Pro Glu Glu Glu Glu Glu
                   165                 170                 175
   Ala Ala Ile Val Glu Pro
               180
<210> 6
   <211> 150
   <212> PRT
   <213> Homo sapiens
<220>
   <221> MISC-FEATURE
   <222> (1)..(150)
   <223> HSPB3
<220>
   <221> DOMAIN
   <222> (64)..(144)
   <223> α-Crystallin domain
<400> 6
   Met Ala Lys Ile Ile Leu Arg His Leu Ile Glu Ile Pro Val Arg Tyr
   1               5                   10                  15
   Gln Glu Glu Phe Glu Ala Arg Gly Leu Glu Asp Cys Arg Leu Asp His
               20                  25                  30
   Ala Leu Tyr Ala Leu Pro Gly Pro Thr Ile Val Asp Leu Arg Lys Thr
           35                  40                  45
   Arg Ala Ala Gln Ser Pro Pro Val Asp Ser Ala Ala Glu Thr Pro Pro
       50                  55                  60
   Arg Glu Gly Lys Ser His Phe Gln Ile Leu Leu Asp Val Val Gln Phe
   65                  70                  75                  80
   Leu Pro Glu Asp Ile Ile Ile Gln Thr Phe Glu Gly Trp Leu Leu Ile
                   85                  90                  95
   Lys Ala Gln His Gly Thr Arg Met Asp Glu His Gly Phe Ile Ser Arg
               100                 105                 110
   Ser Phe Thr Arg Gln Tyr Lys Leu Pro Asp Gly Val Glu Ile Lys Asp
           115                 120                 125
   Leu Ser Ala Val Leu Cys His Asp Gly Ile Leu Val Val Glu Val Lys
       130                 135                 140
   Asp Pro Val Gly Thr Lys
   145                 150
<210> 7
   <211> 160
   <212> PRT
   <213> Homo sapiens
<220>
   <221> MISC-FEATURE
   <222> (1)..(160)
   <223> HSPB6
<220>
   <221> DOMAIN
   <222> (67)..(144)
   <223> α-Crystallin domain
<400> 7
   Met Glu Ile Pro Val Pro Val Gln Pro Ser Trp Leu Arg Arg Ala Ser
   1               5                   10                  15
   Ala Pro Leu Pro Gly Leu Ser Ala Pro Gly Arg Leu Phe Asp Gln Arg
               20                  25                  30
   Phe Gly Glu Gly Leu Leu Glu Ala Glu Leu Ala Ala Leu Cys Pro Thr
           35                  40                  45
   Thr Leu Ala Pro Tyr Tyr Leu Arg Ala Pro Ser Val Ala Leu Pro Val
       50                  55                  60
   Ala Gln Val Pro Thr Asp Pro Gly His Phe Ser Val Leu Leu Asp Val
   65                  70                  75                  80
   Lys His Phe Ser Pro Glu Glu Ile Ala Val Lys Val Val Gly Glu His
                   85                  90                  95
   Val Glu Val His Ala Arg His Glu Glu Arg Pro Asp Glu His Gly Phe
               100                 105                 110
   Val Ala Arg Glu Phe His Arg Arg Tyr Arg Leu Pro Pro Gly Val Asp
           115                 120                 125
   Pro Ala Ala Val Thr Ser Ala Leu Ser Pro Glu Gly Val Leu Ser Ile
       130                 135                 140
   Gln Ala Ala Pro Ala Ser Ala Gln Ala Pro Pro Pro Ala Ala Ala Lys
   145                 150                 155                 160
<210> 8
   <211> 170
   <212> PRT
   <213> Homo sapiens
<220>
   <221> MISC-FEATURE
   <222> (1)..(170)
   <223> HSPB7
<220>
   <221> DOMAIN
   <222> (75)..(152)
   <223> α-Crystallin domain
<400> 8
   Met Ser His Arg Thr Ser Ser Thr Phe Arg Ala Glu Arg Ser Phe His
   1 5 10 15
   Ser Ser Ser Ser Ser Ser Ser Ser Ser Thr Ser Ser Ser Ala Ser Arg
   20 25 30
   Ala Leu Pro Ala Gln Asp Pro Pro Met Glu Lys Ala Leu Ser Met Phe
   35 40 45
   Ser Asp Asp Phe Gly Ser Phe Met Arg Pro His Ser Glu Pro Leu Ala
   50 55 60
   Phe Pro Ala Arg Pro Gly Gly Ala Gly Asn Ile Lys Thr Leu Gly Asp
   65 70 75 80
   Ala Tyr Glu Phe Ala Val Asp Val Arg Asp Phe Ser Pro Glu Asp Ile
   85 90 95
   Ile Val Thr Thr Ser Asn Asn His Ile Glu Val Arg Ala Glu Lys Leu
   100 105 110
   Ala Ala Asp Gly Thr Val Met Asn Thr Phe Ala His Lys Cys Gln Leu
   115 120 125
   Pro Glu Asp Val Asp Pro Thr Ser Val Thr Ser Ala Leu Arg Glu Asp
   130 135 140
   Gly Ser Leu Thr Ile Arg Ala Arg Arg His Pro His Thr Glu His Val
   145 150 155 160
   Gln Gln Thr Phe Arg Thr Glu Ile Lys Ile
   165 170
<210> 9
   <211> 196
   <212> PRT
   <213> Homo sapiens
<220>
   <221> MISC-FEATURE
   <222> (1)..(196)
   <223> HSPB8
<220>
   <221> DOMAIN
   <222> (89)..(169)
   <223> α-Crystallin domain
<400> 9
<210> 10
   <211> 159
   <212> PRT
   <213> Homo sapiens
<220>
   <221> MISC-FEATURE
   <222> (1)..(159)
   <223> HSPB9
<220>
   <221> DOMAIN
   <222> (47)..(130)
   <223> α-Crystallin domain
<400> 10
   Met Gln Arg Val Gly Asn Thr Phe Ser Asn Glu Ser Arg Val Ala Ser
   1               5                   10                  15
   Arg Cys Pro Ser Val Gly Leu Ala Glu Arg Asn Arg Val Ala Thr Met
               20                  25                  30
   Pro Val Arg Leu Leu Arg Asp Ser Pro Ala Ala Gln Glu Asp Asn Asp
           35                  40                  45
   His Ala Arg Asp Gly Phe Gln Met Lys Leu Asp Ala His Gly Phe Ala
       50                  55                  60
   Pro Glu Glu Leu Val Val Gln Val Asp Gly Gln Trp Leu Met Val Thr
   65                  70                  75                  80
   Gly Gln Gln Gln Leu Asp Val Arg Asp Pro Glu Arg Val Ser Tyr Arg
                   85                  90                  95
   Met Ser Gln Lys Val His Arg Lys Met Leu Pro Ser Asn Leu Ser Pro
               100                 105                 110
   Thr Ala Met Thr Cys Cys Leu Thr Pro Ser Gly Gln Leu Trp Val Arg
           115                 120                 125
   Gly Gln Cys Val Ala Leu Ala Leu Pro Glu Ala Gln Thr Gly Pro Ser
       130                 135                 140
   Pro Arg Leu Gly Ser Leu Gly Ser Lys Ala Ser Asn Leu Thr Arg
   145                 150                 155
<210> 11
   <211> 250
   <212> PRT
   <213> Homo sapiens
<220>
   <221> MISC-FEATURE
   <222> (1)..(250)
   <223> HSPB10
<220>
   <221> DOMAIN
   <222> (108)..(201)
   <223> α-Crystallin domain
<400> 11
   Met Ala Ala Leu Ser Cys Leu Leu Asp Ser Val Arg Arg Asp Ile Lys
   1               5                   10                  15
   Lys Val Asp Arg Glu Leu Arg Gln Leu Arg Cys Ile Asp Glu Phe Ser
               20                  25                  30
   Thr Arg Cys Leu Cys Asp Leu Tyr Met His Pro Tyr Cys Cys Cys Asp
           35                  40                  45
   Leu His Pro Tyr Pro Tyr Cys Leu Cys Tyr Ser Lys Arg Ser Arg Ser
       50                  55                  60
   Cys Gly Leu Cys Asp Leu Tyr Pro Cys Cys Leu Cys Asp Tyr Lys Leu
   65                  70                  75                  80
   Tyr Cys Leu Arg Pro Ser Leu Arg Ser Leu Glu Arg Lys Ala Ile Arg
                   85                  90                  95
   Ala Ile Glu Asp Glu Lys Arg Glu Leu Ala Lys Leu Arg Arg Thr Thr
               100                 105                 110
   Asn Arg Ile Leu Ala Ser Ser Cys Cys Ser Ser Asn Ile Leu Gly Ser
           115                 120                 125
   Val Asn Val Cys Gly Phe Glu Pro Asp Gln Val Lys Val Arg Val Lys
       130                 135                 140
   Asp Gly Lys Val Cys Val Ser Ala Glu Arg Glu Asn Arg Tyr Asp Cys
   145                 150                 155                 160
   Leu Gly Ser Lys Lys Tyr Ser Tyr Met Asn Ile Cys Lys Glu Phe Ser
                   165                 170                 175
   Leu Pro Pro Cys Val Asp Glu Lys Asp Val Thr Tyr Ser Tyr Gly Leu
               180                 185                 190
   Gly Ser Cys Val Lys Ile Glu Ser Pro Cys Tyr Pro Cys Thr Ser Pro
           195                 200                 205
   Cys Ser Pro Cys Ser Pro Cys Ser Pro Cys Asn Pro Cys Ser Pro Cys
       210                 215                 220
   Asn Pro Cys Ser Pro Tyr Asp Pro Cys Asn Pro Cys Tyr Pro Cys Gly
   225                 230                 235                 240
   Ser Arg Phe Ser Cys Arg Lys Met Ile Leu
                   245                 250
<210> 12
   <211> 81
   <212> PRT
   <213> Homo sapiens
<220>
   <221> MISC-FEATURE
   <222> (1)..(81)
   <223> HSPB1 α-Crystallin domain
<400> 12
   Ile Arg His Thr Ala Asp Arg Trp Arg Val Ser Leu Asp Val Asn His
   1               5                   10                  15
   Phe Ala Pro Asp Glu Leu Thr Val Lys Thr Lys Asp Gly Val Val Glu
               20                  25                  30
   Ile Thr Gly Lys His Glu Glu Arg Gln Asp Glu His Gly Tyr Ile Ser
           35                  40                  45
   Arg Cys Phe Thr Arg Lys Tyr Thr Leu Pro Pro Gly Val Asp Pro Thr
       50                  55                  60
   Gln Val Ser Ser Ser Leu Ser Pro Glu Gly Thr Leu Thr Val Glu Ala
   65                  70                  75                  80
   Pro
<210> 13
   <211> 81
   <212> PRT
   <213> Homo sapiens
<220>
   <221> MISC-FEATURE
   <222> (1)..(81)
   <223> HSPB2 α-Crystallin domain
<400> 13
   Leu Arg Leu Ser Glu Gly Lys Phe Gln Ala Phe Leu Asp Val Ser His
   1               5                   10                  15
   Phe Thr Pro Asp Glu Val Thr Val Arg Thr Val Asp Asn Leu Leu Glu
               20                  25                  30
   Val Ser Ala Arg His Pro Gln Arg Leu Asp Arg His Gly Phe Val Ser
           35                  40                  45
   Arg Glu Phe Cys Arg Thr Tyr Val Leu Pro Ala Asp Val Asp Pro Trp
       50                  55                  60
   Arg Val Arg Ala Ala Leu Ser His Asp Gly Ile Leu Asn Leu Glu Ala
   65                  70                  75                  80
   Pro
<210> 14
   <211> 77
   <212> PRT
   <213> Homo sapiens
<220>
   <221> MISC-FEATURE
   <222> (1)..(77)
   <223> HSPB5 α-Crystallin domain fragment
<400> 14
   Arg Leu Glu Lys Asp Arg Phe Ser Val Asn Leu Asp Val Lys His Phe
   1               5                   10                  15
   Ser Pro Glu Glu Leu Lys Val Lys Val Leu Gly Asp Val Ile Glu Val
               20                  25                  30
   His Gly Lys His Glu Glu Arg Gln Asp Glu His Gly Phe Ile Ser Arg
           35                  40                  45
   Glu Phe His Arg Lys Tyr Arg Ile Pro Ala Asp Val Asp Pro Leu Thr
       50                  55                  60
   Ile Thr Ser Ser Leu Ser Ser Asp Gly Val Leu Thr Val
   65                  70                  75
<210> 15
   <211> 77
   <212> PRT
   <213> Homo sapiens
<220>
   <221> MISC-FEATURE
   <222> (1)..(77)
   <223> HSPB3 α-Crystallin domain fragment
<400> 15
   Arg Glu Gly Lys Ser His Phe Gln Ile Leu Leu Asp Val Val Gln Phe
   1               5                   10                  15
   Leu Pro Glu Asp Ile Ile Ile Gln Thr Phe Glu Gly Trp Leu Leu Ile
               20                  25                  30
   Lys Ala Gln His Gly Thr Arg Met Asp Glu His Gly Phe Ile Ser Arg
           35                  40                  45
   Ser Phe Thr Arg Gln Tyr Lys Leu Pro Asp Gly Val Glu Ile Lys Asp
       50                  55                  60
   Leu Ser Ala Val Leu Cys His Asp Gly Ile Leu Val Val
   65                  70                  75
<210> 16
   <211> 81
   <212> PRT
   <213> Homo sapiens
<220>
   <221> MISC-FEATURE
   <222> (1)..(81)
   <223> HSPB4 α-Crystallin domain
<400> 16
   Val Arg Ser Asp Arg Asp Lys Phe Val Ile Phe Leu Asp Val Lys His
   1               5                   10                  15
   Phe Ser Pro Glu Asp Leu Thr Val Lys Val Gln Asp Asp Phe Val Glu
               20                  25                  30
   Ile His Gly Lys His Asn Glu Arg Gln Asp Asp His Gly Tyr Ile Ser
           35                  40                  45
   Arg Glu Phe His Arg Arg Tyr Arg Leu Pro Ser Asn Val Asp Gln Ser
       50                  55                  60
   Ala Leu Ser Cys Ser Leu Ser Ala Asp Gly Met Leu Thr Phe Cys Gly
   65                  70                  75                  80
   Pro
<210> 17
   <211> 75
   <212> PRT
   <213> Homo sapiens
<220>
   <221> MISC-FEATURE
   <222> (1)..(75)
   <223> HSPB5 α-Crystallin domain fragment
<400> 17
   Glu Lys Asp Arg Phe Ser Val Asn Leu Asp Val Lys His Phe Ser Pro
   1               5                   10                  15
   Glu Glu Leu Lys Val Lys Val Leu Gly Asp Val Ile Glu Val His Gly
               20                  25                  30
   Lys His Glu Glu Arg Gln Asp Glu His Gly Phe Ile Ser Arg Glu Phe
           35                  40                  45
   His Arg Lys Tyr Arg Ile Pro Ala Asp Val Asp Pro Leu Thr Ile Thr
       50                  55                  60
   Ser Ser Leu Ser Ser Asp Gly Val Leu Thr Val
   65                  70                  75
<210> 18
   <211> 75
   <212> PRT
   <213> Homo sapiens
<220>
   <221> MISC-FEATURE
   <222> (1)..(75)
   <223> HSPB6 α-Crystallin domain fragment
<400> 18
   Asp Pro Gly His Phe Ser Val Leu Leu Asp Val Lys His Phe Ser Pro
   1               5                   10                  15
   Glu Glu Ile Ala Val Lys Val Val Gly Glu His Val Glu Val His Ala
               20                  25                  30
   Arg His Glu Glu Arg Pro Asp Glu His Gly Phe Val Ala Arg Glu Phe
           35                  40                  45
   His Arg Arg Tyr Arg Leu Pro Pro Gly Val Asp Pro Ala Ala Val Thr
       50                  55                  60
   Ser Ala Leu Ser Pro Glu Gly Val Leu Ser Ile
   65                  70                  75
<210> 19
   <211> 73
   <212> PRT
   <213> Homo sapiens
<220>
   <221> MISC-FEATURE
   <222> (1)..(73)
   <223> HSPB5 α-Crystallin domain fragment
<400> 19
   Asp Arg Phe Ser Val Asn Leu Asp Val Lys His Phe Ser Pro Glu Glu
   1               5                   10                  15
   Leu Lys Val Lys Val Leu Gly Asp Val Ile Glu Val His Gly Lys His
               20                  25                  30
   Glu Glu Arg Gln Asp Glu His Gly Phe Ile Ser Arg Glu Phe His Arg
           35                  40                  45
   Lys Tyr Arg Ile Pro Ala Asp Val Asp Pro Leu Thr Ile Thr Ser Ser
       50                  55                  60
   Leu Ser Ser Asp Gly Val Leu Thr Val
   65                  70
<210> 20
   <211> 70
   <212> PRT
   <213> Homo sapiens
<220>
   <221> MISC-FEATURE
   <222> (1)..(70)
   <223> HSPB7 α-Crystallin domain fragment
<400> 20
   Asp Ala Tyr Glu Phe Ala Val Asp Val Arg Asp Phe Ser Pro Glu Asp
   1               5                   10                  15
   Ile Ile Val Thr Thr Ser Asn Asn His Ile Glu Val Arg Ala Glu Lys
               20                  25                  30
   Leu Ala Ala Asp Gly Thr Val Met Asn Thr Phe Ala His Lys Cys Gln
           35                  40                  45
   Leu Pro Glu Asp Val Asp Pro Thr Ser Val Thr Ser Ala Leu Arg Glu
       50                  55                  60
   Asp Gly Ser Leu Thr Ile
   65                  70
<210> 21
   <211> 74
   <212> PRT
   <213> Homo sapiens
<220>
   <221> MISC-FEATURE
   <222> (1)..(74)
   <223> HSPB5 α-Crystallin domain fragment
<400> 21
   Phe Ser Val Asn Leu Asp Val Lys His Phe Ser Pro Glu Glu Leu Lys
   1               5                   10                  15
   Val Lys Val Leu Gly Asp Val Ile Glu Val His Gly Lys His Glu Glu
               20                  25                  30
   Arg Gln Asp Glu His Gly Phe Ile Ser Arg Glu Phe His Arg Lys Tyr
           35                  40                  45
   Arg Ile Pro Ala Asp Val Asp Pro Leu Thr Ile Thr Ser Ser Leu Ser
       50                  55                  60
   Ser Asp Gly Val Leu Thr Val Asn Gly Pro
   65                  70
<210> 22
   <211> 74
   <212> PRT
   <213> Homo sapiens
<220>
   <221> FISC-FEATURE
   <222> (1)..(74)
   <223> HSPB8 α-Crystallin domain fragment
<400> 22
   Trp Lys Val Cys Val Asn Val His Ser Phe Lys Pro Glu Glu Leu Met
   1               5                   10                  15
   Val Lys Thr Lys Asp Gly Tyr Val Glu Val Ser Gly Lys His Glu Glu
               20                  25                  30
   Lys Gln Gln Glu Gly Gly Ile Val Ser Lys Asn Phe Thr Lys Lys Ile
           35                  40                  45
   Gln Leu Pro Ala Glu Val Asp Pro Val Thr Val Phe Ala Ser Leu Ser
       50                  55                  60
   Pro Glu Gly Leu Leu Ile Ile Glu Ala Pro
   65                  70
<210> 23
   <211> 76
   <212> PRT
   <213> Homo sapiens
<220>
   <221> MISC-FEATURE
   <222> (1)..(76)
   <223> HSPB5 α-Crystallin domain fragment
<400> 23
   Lys Asp Arg Phe Ser Val Asn Leu Asp Val Lys His Phe Ser Pro Glu
   1               5                   10                  15
   Glu Leu Lys Val Lys Val Leu Gly Asp Val Ile Glu Val His Gly Lys
               20                  25                  30
   His Glu Glu Arg Gln Asp Glu His Gly Phe Ile Ser Arg Glu Phe His
           35                  40                  45
   Arg Lys Tyr Arg Ile Pro Ala Asp Val Asp Pro Leu Thr Ile Thr Ser
       50                  55                  60
   Ser Leu Ser Ser Asp Gly Val Leu Thr Val Asn Gly
   65                  70                  75
<210> 24
   <211> 79
   <212> PRT
   <213> Homo sapiens
<220>
   <221> MISC-FEATURE
   <222> (1)..(79)
   <223> HSPB9 α-Crystallin domain fragment
<400> 24
   Arg Asp Gly Phe Gln Met Lys Leu Asp Ala His Gly Phe Ala Pro Glu
   1               5                   10                  15
   Glu Leu Val Val Gln Val Asp Gly Gln Trp Leu Met Val Thr Gly Gln
               20                  25                  30
   Gln Gln Leu Asp Val Arg Asp Pro Glu Arg Val Ser Tyr Arg Met Ser
           35                  40                  45
   Gln Lys Val His Arg Lys Met Leu Pro Ser Asn Leu Ser Pro Thr Ala
       50                  55                  60
   Met Thr Cys Cys Leu Thr Pro Ser Gly Gln Leu Trp Val Arg Gly
   65                  70                  75
<210> 25
   <211> 71
   <212> PRT
   <213> Homo sapiens
<220>
   <221> MISC-FEATURE
   <222> (1)..(73)
   <223> HSPB5 α-Crystallin domain fragment
<400> 25
   Asn Leu Asp Val Lys His Phe Ser Pro Glu Glu Leu Lys Val Lys Val
   1               5                   10                  15
   Leu Gly Asp Val Ile Glu Val His Gly Lys His Glu Glu Arg Gln Asp
               20                  25                  30
   Glu His Gly Phe Ile Ser Arg Glu Phe His Arg Lys Tyr Arg Ile Pro
           35                  40                  45
   Ala Asp Val Asp Pro Leu Thr Ile Thr Ser Ser Leu Ser Ser Asp Gly
       50                  55                  60
   Val Leu Thr Val Asn Gly Pro
   65                  70
<210> 26
   <211> 74
   <212> PRT
   <213> Homo sapiens
<220>
   <221> MISC-FEATURE
   <222> (1)..(73)
   <223> HSPB10 α-Crystallin domain fragment
<400> 26
   Ser Val Asn Val Cys Gly Phe Glu Pro Asp Gln Val Lys Val Arg Val
   1               5                   10                  15
   Lys Asp Gly Lys Val Cys Val Ser Ala Glu Arg Glu Asn Arg Tyr Asp
               20                  25                  30
   Cys Leu Gly Ser Lys Lys Tyr Ser Tyr Met Asn Ile Cys Lys Glu Phe
           35                  40                  45
   Ser Leu Pro Pro Cys Val Asp Glu Lys Asp Val Thr Tyr Ser Tyr Gly
       50                  55                  60
   Leu Gly Ser Cys Val Lys Ile Glu Ser Pro
   65                  70